(19) Europäisches Patentamt
European Patent Office
Office européen des brevets

(11) Veröffentlichungsnummer: **0 126 713**
**B1**

(12) **EUROPÄISCHE PATENTSCHRIFT**

(45) Veröffentlichungstag der Patentschrift: **18.01.89**

(21) Anmeldenummer: **84810233.1**

(22) Anmeldetag: **14.05.84**

(51) Int. Cl.⁴: **C 07 C 69/757**, C 07 C 67/30, C 07 C 103/737, C 07 C 103/19, C 07 C 102/00, C 07 C 83/08, C 07 D 295/18, A 01 N 37/08, A 01 N 37/18

(54) **Cyclohexandion-carbonsäurederivate mit herbizider und das Pflanzenwachstum regulierender Wirkung.**

(30) Priorität: **18.05.83 CH 2693/83**
**19.12.83 CH 6747/83**

(43) Veröffentlichungstag der Anmeldung: **28.11.84 Patentblatt 84/48**

(45) Bekanntmachung des Hinweises auf die Patenterteilung: **18.01.89 Patentblatt 89/3**

(84) Benannte Vertragsstaaten: **AT BE CH DE FR GB IT LI LU NL SE**

(56) Entgegenhaltungen:
**EP-A- 0 123 001**
**CH-A- 558 319**
**DE-A- 2 039 466**
**DE-A- 2 232 730**
**JP-A-83 164 543**

(73) Patentinhaber: **CIBA-GEIGY AG, Klybeckstrasse 141, CH-4002 Basel (CH)**

(72) Erfinder: **Brunner, Hans-Georg, Dr., Wannenstrasse 14, CH-4415 Lausen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft neue Cyclohexandion-carbonsäurederivate mit herbizider und das Pflanzenwachstum regulierender Wirkung, Mittel welche diese Cyclohexandioncarbonsäurederivate enthalten sowie die Verwendung dieser Derivate zur selektiven und totalen Unkrautbekämpfung und zur Regulierung des Pflanzenwachstums.

Die neuen Cyclohexandioncarbonsäurederivate entsprechen der Formel I

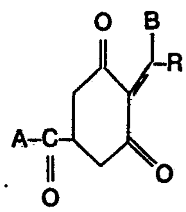

(I),

worin

A einen Rest $-OR_2$ oder $-NR_3R_4$,

B Hydroxyl, einen Rest $-NHOR_1$

R $C_1$–$C_6$ Alkyl oder $C_3$–$C_6$ Cycloalkyl, unsubstituiert oder substituiert durch Halogen, $C_1$–$C_4$ Alkoxy oder $C_1$–$C_4$ Alkylthio,

$R_1$ $C_1$–$C_6$ Alkyl, $C_1$–$C_6$-Halogenalkyl, $C_3$–$C_6$ Alkenyl, $C_3$–$C_6$-Halogenalkenyl oder $C_3$–$C_6$ Alkinyl,

$R_2$, $R_3$ und $R_4$ unabhängig voneinander je Wasserstoff; $C_1$–$C_6$ Alkyl, $C_1$–$C_6$ Halogenalkyl, $C_2$–$C_{10}$ Alkoxyalkyl, $C_2$–$C_{10}$ Alkylthioalkyl; $C_3$–$C_6$ Alkenyl, unsubstituiert oder substituiert durch Halogen, $C_1$–$C_4$ Alkoxy oder $C_1$–$C_4$ Alkylthio; $C_3$–$C_6$ Alkinyl; Phenyl oder $C_1$–$C_6$ Aralkyl, wobei der Phenylkern unsubstituiert oder durch Halogen, $C_1$–$C_4$ Alkyl, $C_1$–$C_4$ Alkoxy, $C_1$–$C_4$ Halogenalkyl, Nitro oder Cyan substituiert ist,

**Beschreibung für die Vertragsstaaten AT, BE, LU, NL, SE**

$R_3$ und $R_4$ zusammen mit dem Stickstoffatom, an das sie gebunden sind, bilden auch einen 5–6 gliedrigen Heterocyclus, der im Ring noch ein Sauerstoff- oder Schwefelatom enthalten kann, bedeuten, oder ein Metall- oder Ammoniumsalz eines solchen Cyclohexandioncarbonsäurederivates.

In diesen Definitionen umfassen die Alkylreste sowohl geradkettige wie verzweigte, z.B. Methyl, Äthyl, Propyl, Isopropyl, Butyl, Isobutyl, sec.Butyl, tert.Butyl sowie alle stereoisomeren Formen der höheren Homologen. Ebenso werden unter Alkenyl und Alkinyl geradkettige wie verzweigte Reste verstanden, z.B. Vinyl, Allyl, Methallyl, Butenyl, Methyl- und Dimethylbutenyl, Äthinyl, Propinyl, Butinyl, Methylbutinyl, Dimethylbutinyl.

Unter Halogen ist Fluor-, Chlor-, Brom- oder Jodatome zu verstehen.

Unter den von $R_3$ und $R_4$ zusammen mit dem Stickstoffatom gebildeten 5–6 gliedrigen Heterocyclen, welche im Ring noch ein Sauerstoff- oder Schwefelatom enthalten können, sind Pyrrol, Pyrrolidin, Piperidin, Morpholin oder auch Thiomorpholin-Reste zu verstehen. Solche Ringe können durch Methylgruppen substituiert sein.

Die Salze dieser Verbindungen werden mit Basen hergestellt. Dazu eignen sich vorzugsweise Alkalimetall-, Erdalkalimetall-, Eisen-, Kupfer-, Nickel-, Zinkhydroxide, aber auch Ammoniak, oder quaternäre $C_1$–$C_4$ Alkyl- oder $C_1$–$C_4$ Hydroxyalkylammonium-Basen.

Die Cyclohexandioncarbonsäurederivate der Formel I zeichnen sich durch gute herbizide und den Pflanzenwuchs regulierende Wirkung aus. Unter den Verbindungen, die durch ihre Wirkung besonders aufgefallen sind, befinden sich folgende Gruppen:

– Cyclohexanderivate der Formel Ia

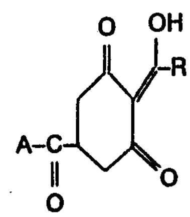

(Ia),

**Beschreibung für die Vertragsstaaten CH, DE, FR, GB, IT, LI**

$R_3$ und $R_4$ zusammen mit dem Stickstoffatom, an das sie gebunden sind, bilden auch einen 5–6 gliedrigen Heterocyclus, der im Ring noch ein Sauerstoff- oder Schwefelatom enthalten kann, bedeuten, oder ein Metall- oder Ammoniumsalz eines solchen Cyclohexandioncarbonsäurederivates, wobei im Falle von Salzen R nicht $C_1$–$C_6$-Alkyl bedeutet, wenn $R_2$ Wasserstoff oder $C_1$–$C_6$-Alkyl ist.

In diesen Definitionen umfassen die Alkylreste sowohl geradkettige wie verzweigte, z.B. Methyl, Äthyl, Propyl, Isopropyl, Butyl, Isobutyl, sec.Butyl, tert.Butyl sowie alle stereoisomeren Formen der höheren Homologen. Ebenso werden unter Alkenyl und Alkinyl geradkettige wie verzweigte Reste verstanden, z.B. Vinyl, Allyl, Methallyl, Butenyl, Methyl- und Dimethylbutenyl, Äthinyl, Propinyl, Butinyl, Methylbutinyl, Dimethylbutinyl.

Unter Halogen ist Fluor-, Chlor-, Brom- oder Jodatome zu verstehen.

Unter den von $R_3$ und $R_4$ zusammen mit dem Stickstoffatom gebildeten 5–6 gliedrigen Heterocyclen, welche im Ring noch ein Sauerstoff- oder Schwefelatom enthalten können, sind Pyrrol, Pyrrolidin, Piperidin, Morpholin oder auch Thiomorpholin-Reste zu verstehen. Solche Ringe können durch Methylgruppen substituiert sein.

Die Salze dieser Verbindungen werden mit Basen hergestellt. Dazu eignen sich vorzugsweise

Alkalimetall-, Erdalkalimetall-, Eisen-, Kupfer-, Nickel-, Zinkhydroxide, aber auch Ammoniak, oder quaternäre $C_1$–$C_4$ Alkyl- oder $C_1$–$C_4$ Hydroxyalkylammonium-Basen.

Die Cyclohexandioncarbonsäurederivate der Formel I zeichnen sich durch gute herbizide und den Pflanzenwuchs regulierende Wirkung aus. Unter den Verbindungen, die durch ihre Wirkung besonders aufgefallen sind, befinden sich folgende Gruppen:

– Cyclohexanderivate der Formel Ia

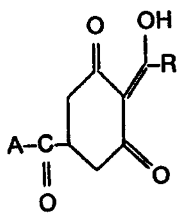

(Ia),

worin A und R die oben gegebene Bedeutung haben, sowie deren Metall- oder Ammoniumsalze.

– Die Derivate der Formel Ia, in denen A einen Rest $-OR_2$ bedeutet und worin R und $R_2$ die oben gegebene Bedeutung haben, sowie deren Metall- oder Ammoniumsalze.

– Die Derivate der Formel Ia, in denen A einen Rest $-NR_3R_4$ bedeutet und worin R, $R_3$ und $R_4$ die oben gegebene Bedeutung haben, sowie deren Metall- und Ammoniumsalze.

– Die Derivate der Formel Ia, in denen A die oben gegebene Bedeutung hat und R einen $C_3$–$C_6$ Cycloalkylrest bedeutet sowie deren Metall- oder Ammoniumsalze.

Ferner die Cyclohexandione der Formel Ib

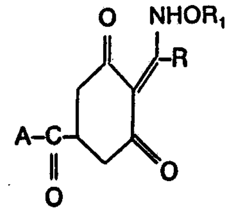

(Ib)

worin A, R und $R_1$ die oben gegebene Bedeutung haben, sowie deren Metall- und Ammoniumsalze.

– Die Derivate der Formel Ib, in denen A einen Rest $-OR_2$ bedeutet und worin R, $R_1$ und $R_2$ die oben gegebene Bedeutung haben, sowie deren Metall- und Ammoniumsalze.

– Die Derivate der Formel Ib, in denen A einen Rest $-NR_3R_4$ bedeutet und worin R, $R_1$, $R_3$ und $R_4$ die oben gegebene Bedeutung haben, sowie deren Metall- und Ammoniumsalze.

Aufgefallen als Einzelverbindungen sind:

4-(1-Hydroxybutyliden)-3,5-cyclohexandion-carbonsäure-äthylester,
4-(1-Hydroxybutyliden)-3,5-cyclohexandicarbonsäure-isobutylester,
4-(1-Äthoxyaminobutyliden)-3,5-cyclohexandion-carbonsäure-äthylester,
4-(1-Allyloxyaminobutyliden)-3,5-cyclohexandion-carbonsäure-äthylester,
4-(Cyclopropyl-hydroxymethyliden)-3,5-cyclo-hexandioncarbonsäure-äthylester,
4-(1-Äthoxyaminobutyliden)-3,5-cyclohexandion-carbonsäure-dimethylamid,
4-(1-Allyloxyaminobutyliden)-3,5-cyclohexandion-carbonsäure-dimethylamid

sowie ihre Natrium-, Ammonium- und Tetramethylammoniumsalze.

Die Cyclohexandioncarbonsäurederivate der Formel I können in verschiedenen tautomeren Formen auftreten. Beispielsweise existieren für den 4-(1-Äthoxyaminobutyliden)-3,5-cyclohexandioncarbonsäuremethylester folgende Formen:

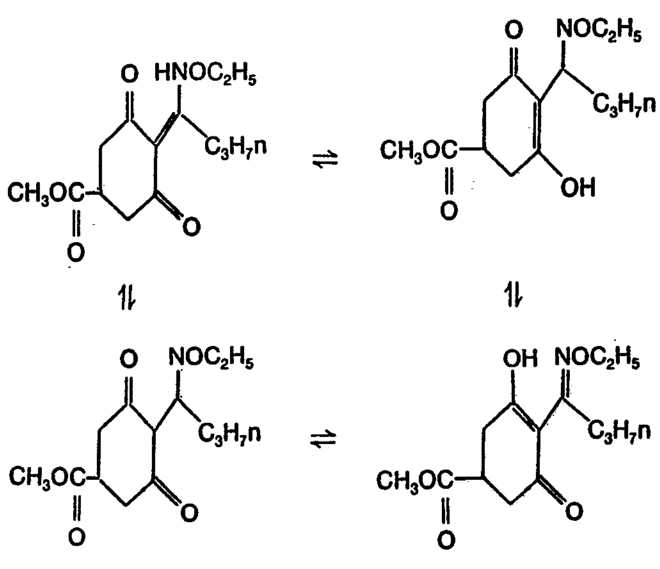

Einige 2-(1-Hydroxyalkyliden)-3,5-cyclohexandion-4-carbonsäuren und -Ester mit herbizider und den Pflanzenwuchs regulierender Wirkung sind bereits in der publizierten Japanischen Patentanmeldung JP-A 164 543/83 beschrieben. Die Salze solcher Säuren, Ester und Thioester und ihre Herstellung sind später noch eingehender in der EP-A-123 001 abgehandelt worden.

Die Cyclohexandioncarbonsäurederivate der Formel I werden auf herkömmliche Weise hergestellt, indem man ein 3,5-Cyclohexandioncarbonsäurederivat der Formel II

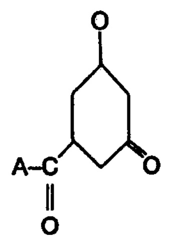

(II),

worin A einen wie oben definierten Ester- oder Amidrest darstellt, in einem inerten organischen Lösungsmittel, in Gegenwart einer Base als säurebindendem Mittel, mit einem Säurehalogenid der Formel III umsetzt

Hal–COR (III),

worin R die oben gegebene Bedeutung hat, das erhaltene Produkt isoliert und gegebenenfalls weiter in einem inerten mit Wasser nicht mischbaren Lösungsmittel bei Siedetemperatur unter wasserabspaltenden Bedingungen mit einem Hydroxylamin der Formel IV umsetzt

$HONHR_1$ (IV),

worin $R_1$ die oben gegebene Bedeutung hat und das erhaltene Produkt isoliert.

Als Lösungsmittel für diese Umsetzungen kommen vor allem Aromaten wie Benzol, Toluol, Xylol, sowie Halogenwasserstoffe wie Chloroform, Dichloräthan, Tetrachlorkohlenstoff in Frage.

Die Reaktionstemperaturen liegen bei Raumtemperatur und dem Siedepunkt des Reaktionsgemisches. Während der Zugabe von Säurechlorid ist möglicherweise eine Kühlung des Reaktionsgefässes angezeigt.

Als säurebindende Mittel kommen organische wie anorganische Basen in Betracht. Beispiele sind Pyridin, 4-Aminopyridin, Kollidin, Triäthylamin, Ammonium-, Natrium-, Kalium- oder Calciumcarbonat oder die entsprechenden Bicarbonate.

Als Säurehalogenide der Formel III kommen hauptsächlich Essigsäurechlorid, Propionsäurechlorid, Buttersäurechlorid, Valeriansäurechlorid, 3-Methoxypropionsäurechlorid, 2-Chlorpropionsäurechlorid, Cyclopropansäurechlorid oder Cyclohexansäurechlorid, wie auch die entsprechenden Bromide in Frage.

Als Hydroxylamine der Formel IV kommen hauptsächlich die Methyl-, Äthyl-, Chloräthyl-, Propyl-, Isopropyl-, Butyl-, Isobutyl-, Allyl-, Cycloallyl-, Methallyl- und Propinyl-hydroxylamine in Frage, welche auch in Salzform, z.B. als Hydrochlorid in die Reaktion gegeben werden können.

Die als Ausgangsstoff benötigten Cyclohexandioncarbonsäurederivate der Formel II werden einerseits durch Hydrieren von 3,5-Dihydroxybenzoesäure mit Wasserstoff und Raney-Nickel und nachheriger Veresterung oder Amidierung des Säurerestes entsprechend dem folgenden Schema erhalten

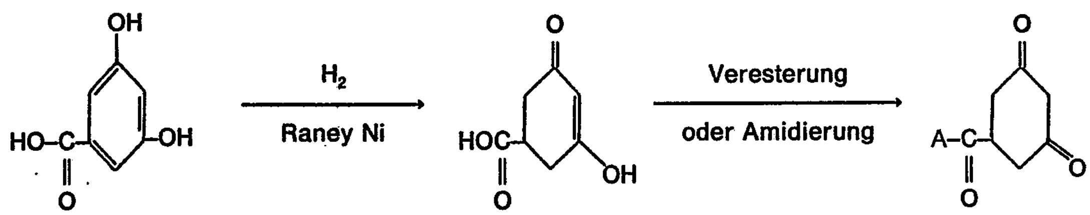


wobei die Ketogruppe gegebenenfalls geschützt werden muss, z.B. als Enoläther oder als Enamin, siehe dazu auch J. Am., Chem. Soc. 78, 4405 (1956).

Man kann aber auch ein 3,5-Dihydroxybenzoesäurederivat mit Wasserstoff und Raney-Nickel hydrieren entsprechend dem Schema

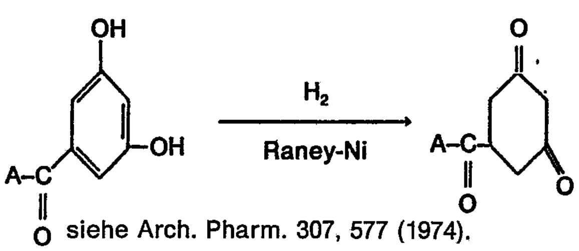


siehe Arch. Pharm. 307, 577 (1974).

Bei geringen Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Zuckerrohr, Getreide, Baumwolle, Soja, Mais und Reis befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen

der Formel I wirken bereits bei – im Vergleich zu anderen herbiziden und Wuchsregulatoren – sehr geringen Aufwandmengen.

Die Verbindungen der Formel I haben ausserdem starke pflanzenwuchsregulierende Eigenschaften, die sich in einer Ertragssteigerung von Kulturpflanzen oder Erntegut auswirken kann. Viele Verbindungen der Formel I zeigen darüberhinaus eine konzentrationsabhängige pflanzenwuchshemmende Wirkung. Es werden sowohl Monokotyledonen als auch Dikotyledonen in ihrem Wachstum beeinträchtigt.

So können z.B. die in der Landwirtschaft in tropischen Gegenden häufig als «cover crops» (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die «cover crops» jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Bei monokotylen Pflanzen, z.B. Gräsern oder auch Kulturpflanzen wie Getreide, ist eine Hemmung des vegetativen Wachstums manchmal gewünscht und vorteilhaft. Eine derartige Wuchshemmung ist unter anderem bei Gräsern von wirtschaftlichem Interesse, denn dadurch kann z.B. die Häufigkeit der Grasschnitte in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Überlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens («Lagerns») der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Weiter eignen sich die Verbindungen der Formel I, um das Keimen von eingelagerten Kartoffeln zu verhindern. Bei Kartoffeln entwickeln sich bei der Einlagerung über den Winter häufig Keime, die Schrumpfen, Gewichtsverlust und Faulen zur Folge haben.

Bei grösseren Aufwandmengen werden alle getesteten Pflanzen in ihrer Entwicklung so geschädigt, dass sie absterben.

Die Erfindung betrifft auch herbizide und pflanzenwuchsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmittel eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$–$C_{22}$), wie z.B. die Na- oder K-Salze der Öl- oder Stearinsäure, oder von natürlichen Fettsäuregemischen, die z.B. aus Kokosnuss- oder Talgöl gewonnen werden können, genannt. Ferner sind auch die Fettsäure-methyltaurinsalze zu erwähnen.

Häufig werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2-Sulfonsäuregruppen und einen Fettsäurerest mit 8–22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Äthylenoxid-Adduktes und Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

«Mc Cutcheon's Detergents and Emulsifiers Annual» MC Publishing Corp., Ridgewood, New Jersey, 1979.

J and M. Ash, «Encyclopedia of Surfactants» Vol. I–III, Chemical Publishing Co., Inc. New York, 1980/81.

Die Wirkstoffzubereitungen enthalten in der Regel 0,1 bis 95%, insbesondere 0,1 bis 80%, Wirkstoff der Formel I, 1 bis 99,9% eines festen oder flüssigen Zusatzstoffes und 0 bis 25%, insbesondere 0,1 bis 25% eines Tensides.

Insbesondere setzen sich bevorzugte Formulierungen folgendermassen zusammen: (% = Gewichtsprozent)

Emulgierbare Konzentrate
Aktiver Wirkstoff:
1 bis 20%, bevorzugt 5 bis 10%
oberflächenaktive Mittel:
5 bis 30%, bevorzugt 10 bis 20%
flüssiges Trägermittel:
50 bis 94%, bevorzugt 70 bis 85%

Stäube
Aktiver Wirkstoff:
0,1 bis 10%, vorzugsweise 0,1 bis 1%
festes Trägermittel:
99,9 bis 90%, vorzugsweise 99,9 bis 99%

Suspensions-Konzentrat
Aktiver Wirkstoff:
5 bis 75%, vorzugsweise 10 bis 50%
Wasser:
94 bis 25%, vorzugsweise 90 bis 30%
oberflächenaktives Mittel:
1 bis 40%, vorzugsweise 2 bis 30%

Benetzbare Pulver
Aktiver Wirkstoff:
0,5 bis 90%, vorzugsweise 1 bis 80%
oberflächenaktives Mittel:
0,5 bis 20%, vorzugsweise 1 bis 15%
festes Trägermittel:
5 bis 95%, vorzugsweise 15 bis 90%

Granulate
Aktiver Wirkstoff:
0,5 bis 30%, vorzugsweise 3 bis 15%
festes Trägermittel:
99,5 bis 70%, vorzugsweise 97 bis 85%

Während als Handelswerte eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,01 bis 10 kg/ha, vorzugsweise 0,025 bis 5 kg/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C, die Drücke in Millibar mb angegeben.

Herstellungsbeispiele:
Beispiel 1:
Herstellung von 4-Buturyl-3,5-cyclohexandioncarbonsäureisobutylester

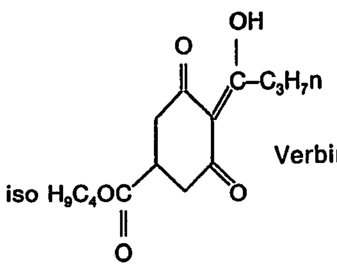

Verbindung 1.3

a) 3,5-Cyclohexandioncarbonsäureisobutylester

Man kocht über Nacht am Wasserabscheider ein Gemisch von 50 g 3,5-Cyclohexandioncarbonsäure, 150 ml Isobutanol, 30 g 85%ige ortho Phosphorsäure und 400 ml Toluol. Die Lösung wird dann am Rotationsverdampfer eingeengt. Der Rückstand wird in 200 ml Tetrahydrofuran aufgenommen, mit 100 ml 1N Salzsäure versetzt und während 2 Stunden gekocht. Nach dem Abkühlen wird Essigester zugegeben. Die organische Phase wird abgetrennt, mit gesättigter Kochsalzlösung gewaschen, getrocknet und eingedampft. Es verbleibt ein wachsartiger Rückstand, der aus Äther/Hexan umkristallisiert wird und bei 74–76° schmilzt.

b) 4-Buturyl-3,5-cyclohexandioncarbonsäureisobutylester

Zu einer Lösung von 60 g 3,5-Cyclohexandioncarbonsäureisobutylester und 25 ml Pyridin in 400 ml Dichloräthan tropft man 30 ml Buttersäurechlorid und rührt bei Raumtemperatur 15 Stunden weiter. Die Reaktionslösung wird dann filtriert, mit 1N Salzsäure gewaschen, getrocknet und eingeengt. Das so erhaltene O-acylierte Produkt wird in 200 ml Dichloräthan aufgenommen, mit 4 g 4-Dimethylaminopyridin versetzt und 4 Stunden am Rückfluss gekocht. Die abgekühlte Reaktionslösung wird dann mit 1N Salzsäure gewaschen, getrocknet, eingeengt und an wenig Kieselgel chromatographiert. Man erhält so 51 g 4-Buturyl-3,5-cyclohexandioncarbonsäure-isobutylester als helles Öl, welches einen Brechungsindex $n_D^{21}$ 1.4907 aufweist.

Beispiel 2:
Herstellung von 4-(1-Allyloxyaminobutyliden)-3,5-cyclohexandioncarbonsäure-isobutylester

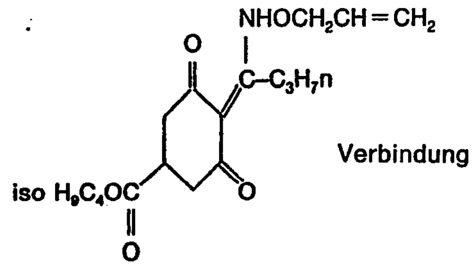

Verbindung 2.5

Ein Gemisch von 14 g 4-Buturyl-3,5-cyclohexandioncarbonsäureisobutylester, 6,5 g O-Allylhydroxylamin-Hydrochlorid, 6,5 g Kaliumcarbonat und 150 ml Chloroform wird während 6 Stunden am Rückfluss gekocht. Die Reaktionslösung wird dann mit 1N Salzsäure gewaschen, getrocknet, eingeengt und an wenig Kieselgel mit Essigester/Petroläther 1:3 chromatographiert. Nach Verdunsten des Lösungsmittels erhält man 6,9 g 4-(1-Allyloxyaminobutyliden)-3,5-cyclohexandioncarbonsäure-isobutylester als helles Öl, welches einen Brechungsindex $n_D^{21}$ 1.4989 aufweist.

Beispiel 3:
Herstellung von 4-(1-Äthoxyaminobutyliden)-3,5-cyclohexandioncarbonsäure-dimethylamid

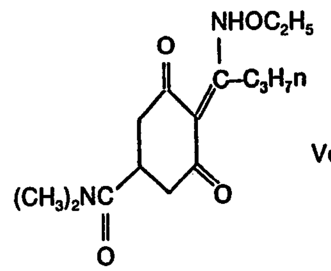

Verbindung 2.72

a) Zu einer Lösung von 15,6 g Cyclohexan-3,5-dioncarbonsäure tropft man unter Rühren 18,4 ml Dimethylcarbamoylchlorid und rührt anschliessend 12 Stunden bei Raumtemperatur und 2 Stunden bei Siedetemperatur unter Rückfluss weiter. Nach dem Abkühlen nimmt man das Reaktionsgemisch in 400 ml Äthylacetat auf, wäscht die organische Phase vier mal mit Salzsole, trocknet über Magnesiumsulfat und engt sie ein. Der Rückstand besteht aus 13,2 g rohem 3-(N,N-Dimethylcarbamoyl)-5-oxo-cyclohex-(3)-en-carbonsäure-dimethylamid. Dieser wird in 300 ml Tetrahydrofuran gelöst, mit 8 ml konzentrierter Salzsäure versehen und 2 Stunden bei Raumtemperatur gerührt. Das Reaktionsgemisch wird dann mit Salzsole gewaschen, über Magnesiumsulfat getrocknet und eingeengt. Es verbleiben 13,2 g 3,5-Cyclohexandioncarbonsäure-dimethylamid als harzartige Substanz, die nach Reinigung durch Chromatographie mit Hexan/Äther über eine Silikagelkolonne kristallin anfällt. Schmelzpunkt 152–155 °C.

b) Die 13,2 g 3,5-Cyclohexandioncarbonsäuredimethylamid werden zusammen mit 6,9 ml Pyridin in 100 ml Äthylenchlorid gelöst. Dann tropft man unter Rühren 8,5 ml Buttersäurechlorid zu dieser Lösung, was eine leicht exotherme Reaktion hervorruft. Die entstandene gelbe Suspension wird während 14 Stunden bei Raumtemperatur gerührt, dann mit 1N Salzsäure und Salzsole gewaschen, über Magnesiumsulfat getrocknet und eingeengt. Der Rückstand wird in 100 ml Dichloräthan gelöst und mit 0,5 g 4-Dimethylaminopyridin und 0,1 ml Buttersäurechlorid während 2 Stunden am Rückfluss gekocht. Nach dem Erkalten wird das Reaktionsgemisch mit 20 ml 1n, mit Kochsalz gesättigter Salzsäure gewaschen, über Magnesiumsulfat getrocknet und eingeengt. Der Rückstand wird zur Reinigung über eine Silikagelkolonne mit Äthylacetat als Laufmittel chromatographiert. Man erhält so 7,8 g 4-Butyryl-3,5-cyclohexandioncarbonsäure-dimethylamid als helles Öl.

c) Ein Gemisch von 4,3 g des oben erhaltenen 4-Butyryl-3-cyclohexandioncarbonsäuredimethylamides, 1,9 g Äthoxyaminhydrochlorid, 1,4 g Kaliumcarbonat in 50 ml Chloroform und 5 ml Methanol wird während 24 Stunden bei Raumtemperatur gerührt. Das Reaktionsgemisch wird dann mit 1N Salzsäure gewaschen, über Magnesiumsulfat getrocknet und eingeengt. Der Rückstand ist ein Öl, welches durch Chromatographie über eine Silikagelsäule mit Äthylacetat als Laufmittel gereinigt wird. Nach Verdampfen des Lösungsmittels verbleibt ein Öl das beim Stehen kristallisiert. Man erhält so 2 g Titelsubstanz vom Schmelzpunkt 54–58 °C.

Beispiel 4:
Herstellung von 3,5 4-(Äthoxyaminobutyliden)-3,5-cyclohexandioncarbonsäure-benzylamid

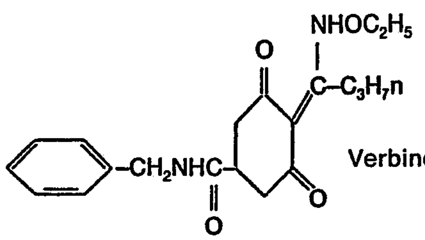

Verbindung 2.47

a) Eine Lösung von 107 g 3,5-Cyclohexandioncarbonsäure und 1 ml konzentrierte Schwefelsäure in 400 ml Methanol wird während 3 Stunden bei Raumtemperatur gerührt. Schon während des Rührens fällt kristallines Material aus. Man gibt 400 ml Äther zu, worauf 3-Methoxy-5-oxo-cyclohex-3-en-carbonsäure kristallin ausfällt. Nach Filtrieren und Trocknen im Exsikkator erhält man 120 g Produkt.

b) Man mischt 34 g 3-Methoxy-5-oxo-cyclohex-3-en-carbonsäure, 33 g N,N'-carbonyldiimidazol und 300 ml Dichloräthan und rührt die Suspension eine Stunde bei Raumtemperatur. Dann tropft man 22 ml Benzylamin zu und lässt weitere 14 Stunden (über Nacht) bei Raumtemperatur weiterrühren. Dann gibt man 1N Salzsäure zum Reaktionsgemisch bis der pH zwischen 3 und 4 ist. Die organische Phase wird abgetrennt, mit Salzsole gewaschen, getrocknet über Magnesiumsulfat eingeengt. Man erhält so 43,6 g 3-Methoxy-5-oxy-cyclohex-3-encarbonsäure-benzylamid als helles Öl.

c) 42 g dieses 3-Methoxy-5-oxy-cyclohex-3-encarbonsäure-benzylamides werden in 500 ml Tetrahydrofuran gelöst. Man gibt 0,5 ml konzentrierte Salzsäure und 10 ml Wasser zu und rührt während 5 Stunden bei Raumtemperatur. Beim Trocknen der Lösung über Molekularsieb kristallisieren 16,6 g 3,5-Cyclohexandion-carbonsäure-benzylamid aus. Weitere 17,8 g konnten durch Einengen der Mutterlauge gewonnen werden. Ausbeute 34,4 g, Schmelzpunkt 178–181°.

d) 29 g 3,5-Cyclohexandion-carbonsäure-benzylamin werden analog Beispiel 3b in Äthylenchlorid in Gegenwart von etwas Pyridin mit Buttersäurechlorid behandelt. Nach dem Aufarbeiten des Reaktionsgemisches wird das 4-Butyryl-3,5-cyclohexandioncarbonsäurebenzylamid als wachsartige Masse isoliert, welche aus Äther/Hexan kristallisiert. Ausbeute 15 g, Schmelzpunkt 126–128°.

e) 6 g 4-Butyryl-3,5-cyclohexandioncarbonsäure-benzylamid werden gemäss Beispiel 3c in Chloroform in Gegenwart von Kaliumcarbonat mit Äthoxyamin-Hydrochlorid umgesetzt. Nach dem Aufarbeiten des Reaktionsgemisches erhält man 2,3 g 4-(1-Äthoxyaminobutyliden)-3,5-cyclohexandioncarbonsäure-benzylamid in kristalliner Form, Schmelzpunkt 88–90°.

In Analogie zu diesen Beispielen werden folgende Verbindungen hergestellt:

Tabelle 1

| No. | A | R | $M^{\oplus}$ | phys. Daten |
|---|---|---|---|---|
| 1.1 | $OCH_3$ | $C_3H_7n$ | | |
| 1.2 | $OC_2H_5$ | $C_3H_7n$ | | $n_D^{27}$ 1.5060 |
| 1.3 | $OC_4H_9$ iso | $C_3H_7n$ | | $n_D^{27}$ 1.4907 Bsp. 1 |
| 1.4 | $OCH_2SCH_3$ | $C_3H_7n$ | | $n_D^{25}$ 1.5193 |
| 1.5 | $OC_2H_4SCH_3$ | $C_3H_7n$ | | |
| 1.6 | $OC_2H_4OCH_3$ | $C_3H_7n$ | | |
| 1.7 | $OC_3H_7i$ | $C_3H_7n$ | | |
| 1.8 | $OC_3H_6Cl$ | $C_3H_7n$ | | $n_D^{25}$ 1.4998 |
| 1.9 | $OCH_2$- 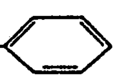 | $C_3H_7n$ | | |

| No. | A | R | $M^{\oplus}$ | phys. Daten |
|---|---|---|---|---|
| 1.10 | 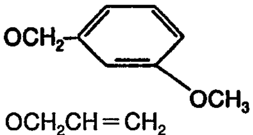 | $C_3H_7n$ | | |
| 1.11 | $OCH_2CH=CH_2$ | $C_3H_7n$ | | |
| 1.12 | $OCH_2CCl=CH_2$ | $C_3H_7n$ | | |
| 1.13 | $OCH_2C\equiv CH$ | $C_3H_7n$ | | |
| 1.14 | $OCH_3$ | $C_6H_{13}n$ | | |
| 1.15 | $OC_2H_5$ | Cyclopropyl | | $n_D^{30}$ 1.5330 |
| 1.16 | $OCH_3$ | Cyclohexyl | | |
| 1.17 | $OC_6H_{13}$ | $CH_3$ | | |
| 1.18 | $OCH_2SCH_3$ | $CH_3$ | | |
| 1.19 | $OC_2H_5$ | $CH_2SCH_3$ | | |
| 1.20 | $OC_2H_5$ | $C_2H_4OCH_3$ | | |
| 1.21 | $OC_4H_9$ tert. | $CHClCH_3$ | | |
| 1.22 | $NH_2$ | $C_3H_7n$ | | Smp. 167–169° |
| 1.23 | $NH_2$ | $C_2H_5n$ | | |
| 1.24 | $N(CH_3)_2$ | $C_3H_7n$ | | |
| 1.25 | $N(CH_3)_2$ | $C_3H_7i$ | | |
| 1.26 | $NHC_4H_9$ iso | $C_3H_7n$ | | Smp. 126–128° |
| 1.27 | $NHC_4H_9$ iso | $CH_3$ | | |
| 1.28 | $N(CH_2\text{–}CH=CH_2)_2$ | Cyclopropyl | | |
| 1.29 | $NHCH_2\text{–}C\equiv CH$ | $CH_2OCH_3$ | | |
| 1.30 | 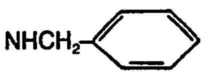 | $C_3H_7n$ | | |
| 1.31 | 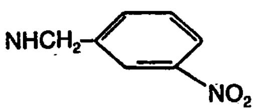 | $C_2H_4Cl$ | | |
| 1.32 | 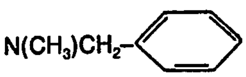 | $C_2H_4OC_2H_5$ | | |
| 1.33 | $NHC_2H_4SC_2H_5$ | $C_4H_9$ iso | | |
| 1.34 | Piperidino | $C_3H_7$ iso | | |
| 1.35 | Morpholino | $C_6H_{13}n$ | | |
| 1.36 | $OC_2H_5$ | $C_3H_7n$ | $Na^+$ | |
| 1.37 | $OC_2H_5$ | $C_3H_7n$ | $NH_4^+$ | |
| 1.38 | $OC_4H_9$ iso | $C_3H_7n$ | ½ $Mg^+$ | |
| 1.39 | $OC_4H_9$ iso | $C_3H_7n$ | ½ $Zn^+$ | |
| 1.40 | $N(CH_3)_3$ | $C_3H_7n$ | $N(CH_3)_4^+$ | |
| 1.41 | $NH_2$ | $C_2H_5$ | $Na^+$ | |
| 1.42 | $OC_2H_5$ | $CH_3$ | | Smp. 49–51° |
| 1.43 | $OC_2H_5$ | Cyclobutyl | | |
| 1.44 | $OCH_3$ | Cyclobutyl | | |
| 1.45 | $OC_2H_5$ | Cyclopentyl | | |
| 1.46 | $OCH_3$ | Cyclopentyl | | |
| 1.47 | $OC_2H_5$ | Cyclohexyl | | |
| 1.48 | $OCH_3$ | Cyclohexyl | | |
| 1.49 | $N(CH_3)_2$ | Cyclopropyl | | Smp. 109–112° (Wachs) |
| 1.50 | 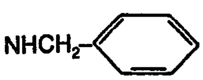 | Cyclopropyl | | Smp. 130–144° Wachs |
| 1.51 | 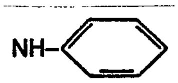 | Cyclopropyl | | |

| No. | A | R | $M^{\oplus}$ | phys. Daten |
|---|---|---|---|---|
| 1.52 | 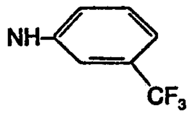 | Cyclopropyl | | |
| 1.53 | $N(CH_3)OCH_3$ | $C_3H_7n$ | | Öl |
| 1.54 | $N(CH_3)OCH_3$ | $C_2H_5$ | | |
| 1.55 | 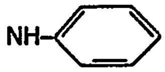 | $C_3H_7n$ | | Smp. 123–127° |
| 1.56 | 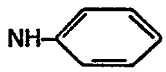 | $CH_3$ | | |
| 1.57 | 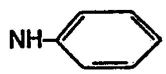 | $C_6H_{13}n$ | | |
| 1.58 | 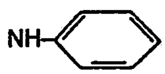 | $C_2H_4CH(CH_3)_2$ | | |
| 1.59 | 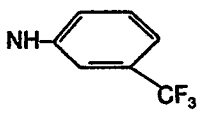 | $C_3H_7n$ | | |
| 1.60 | 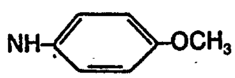 | $C_3H_7n$ | | |
| 1.61 | 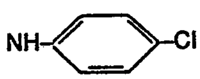 | $CH_3$ | | |
| 1.62 | $N(C_2H_5)_2$ | $C_3H_7n$ | | Öl |
| 1.63 | $NHCH_3$ | $C_3H_7n$ | | |
| 1.64 | 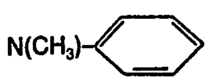 | $C_3H_7n$ | | |
| 1.65 | Cyclopropylamino | $C_3H_7n$ | | |
| 1.66 | $NHC_6H_{13}n$ | $C_3H_7n$ | | |
| 1.67 | $NHC_2H_4SCH_3$ | $C_3H_7n$ | | Wachs |
| 1.68 | $NHC_2H_4SCH_3$ | $C_2H_5$ | | |
| 1.69 | $NHC_2H_4SC_3H_7i$ | $C_3H_7n$ | | |
| 1.70 | 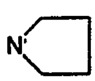 | $C_3H_7n$ | | Wachs |
| 1.71 | $N(CH_2CH=CH_2)_2$ | $C_3H_7n$ | | Öl |
| 1.72 | $N(CH_3)_2$ | Cyclobutyl | | |
| 1.73 | $N(CH_3)_2$ | $C_6H_{13}n$ | | |
| 1.74 | $N(CH_3)_2$ | $C_2H_5$ | | Smp. 82–83° |
| 1.75 | $NHCH_3$ | $C_5H_{11}n$ | | |
| 1.76 | $N(CH_3)C_2H_5$ | $C_3H_7n$ | | |
| 1.77 | $N(CH_3)C_2H_5$ | $C_3H_7i$ | | |
| 1.78 | $N(CH_3)C_2H_5$ | Cyclopropyl | | |
| 1.79 | $N(CH_3)_2$ | $C_5H_{11}n$ | | Smp. 70–72° |

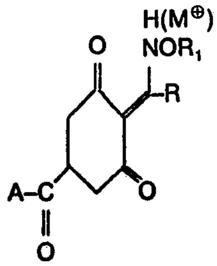

Tabelle 2

| No. | A | R | $R_1$ | $M^{\oplus}$ | phys. Daten |
|---|---|---|---|---|---|
| 2.1 | $OCH_3$ | $C_3H_7n$ | $C_2H_5$ | | |
| 2.2 | $OC_2H_5$ | $C_3H_7n$ | $C_2H_5$ | | $n_D^{27}$ 1.5002 |
| 2.3 | $OC_2H_5$ | $C_3H_7n$ | $CH_2CH=CH_2$ | | $n_D^{27}$ 1.5112 |
| 2.4 | $OC_4H_9$ iso | $C_3H_7n$ | $C_2H_5$ | | $n_D^{27}$ 1.4929 |
| 2.5 | $OC_4H_9$ iso | $C_3H_7n$ | $CH_2CH=CH_2$ | | $n_D^{27}$ 1.4989 Beispiel 2 |
| 2.6 | $OCH_2SCH_3$ | $C_3H_7n$ | $C_2H_5$ | | $n_D^{25}$ 1.5198 |
| 2.7 | $OCH_2SCH_3$ | $C_3H_7n$ | $CH_2-C\equiv CH$ | | |
| 2.8 | $OC_3H_7$ iso | $C_3H_7n$ | $C_2H_5$ | | $n_D^{25}$ 1.5003 |
| 2.9 | $OC_3H_7$ iso | $C_3H_7n$ | $CH_2-CH=CH_2$ | | $n_D^{25}$ 1.5088 |
| 2.10 | $OC_3H_7$ iso | $C_3H_7n$ | $CH_2CCl=CH_2$ | | |
| 2.11 | $OC_2H_4SCH_3$ | $C_3H_7n$ | $C_2H_5$ | | |
| 2.12 | $OC_2H_4SCH_3$ | $C_3H_7n$ | $CH_2-CH=CH_2$ | | |
| 2.13 | $OC_2H_4SCH_3$ | $C_3H_7n$ | $C_2H_4Cl$ | | |
| 2.15 | $OC_2H_4OCH_3$ | $C_3H_7n$ | $C_2H_5$ | | |
| 2.16 | $OC_3H_6Cl$ | $C_3H_7n$ | $C_2H_5$ | | |
| 2.17 | 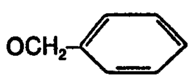 | $C_3H_7n$ | $C_2H_5$ | | |
| 2.18 | 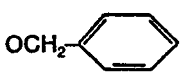 | $C_3H_7n$ | $CH_2-CH=CH_2$ | | |
| 2.19 | 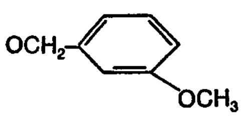 | $C_3H_7n$ | $C_6H_{13}n$ | | |
| 2.20 | $OCH_2CH=CH_2$ | $C_3H_7n$ | $C_6H_{13}n$ | | |
| 2.21 | $OCH_2-CH=CH_2$ | $C_3H_7n$ | $CH_3$ | | |
| 2.22 | $OC_2H_4Cl$ | $C_3H_7n$ | $CH_2-C\equiv CH_2$ | | |
| 2.23 | $OCH_2CCl=CH_2$ | $C_3H_7n$ | $C_2H_4Cl$ | | |
| 2.24 | $OCH_2-C\equiv CH$ | $C_3H_7n$ | $C_6H_{13}$ iso | | |
| 2.25 | $OCH_3$ | $C_6H_{13}n$ | $C_2H_5$ | | |
| 2.26 | $OC_2H_5$ | Cyclopropyl | $CH_2-CH=CH_2$ | | |
| 2.27 | $OCH_3$ | Cyclohexyl | $C_2H_5$ | | |
| 2.28 | $OC_6H_{13}n$ | $CH_3$ | $C_4H_9n$ | | |
| 2.29 | $OCH_3SCH_3$ | $CH_3$ | $C_4H_9$ sec | | |
| 2.30 | $OC_2H_5$ | $CH_2SCH_3$ | $C_2H_5$ | | |
| 2.31 | $OC_2H_5$ | $C_2H_4OCH_3$ | $CH_2-C\equiv CH$ | | |
| 2.32 | $OC_4H_9$ tert. | $CHCl-CH_3$ | $C_2H_5$ | | |
| 2.33 | $NH_2$ | $C_3H_7n$ | $C_2H_5$ | | |
| 2.34 | $NH_2$ | $C_3H_7n$ | $CH_2-CH=CH_2$ | | Smp. 127–129° |
| 2.35 | $NH_2$ | $C_2H_5$ | $C_2H_5$ | | |
| 2.36 | $NH_2$ | $C_2H_5$ | $CH_2-CCl=CH_2$ | | |
| 2.37 | $N(CH_3)_2$ | $C_3H_7n$ | $C_2H_5$ | | Smp. 54–58° |
| 2.38 | $N(CH_3)_2$ | $C_3H_7n$ | $CH_2-CH=CH_2$ | | Smp. 59–65° |
| 2.39 | $N(CH_3)_2$ | $C_3H_7i$ | $CH_3$ | | |
| 2.40 | $N(CH_3)_2$ | $C_3H_7i$ | $C_3H_6Br$ | | |
| 2.41 | $NHC_4H_9$ iso | $C_3H_7n$ | $C_2H_5$ | | Smp. 88–90° |
| 2.42 | $NHC_4H_9$ iso | $C_3H_7n$ | $CH_3$ | | |

| No. | A | R | $R_1$ | $M^{\oplus}$ | phys. Daten |
|---|---|---|---|---|---|
| 2.43 | $NHC_4H_9$ iso | $C_3H_7n$ | $CH_2CH=CH_2$ | | Smp. 100–102° |
| 2.44 | $NHC_4H_9$ iso | $CH_3$ | $CH_2C\equiv CH$ | | |
| 2.45 | $N(CH_2\text{-}CH=CH_2)_2$ | Cyclopropyl | $C_2H_5$ | | |
| 2.46 | $NHCH_2\text{-}C\equiv CH$ | $CH_2OCH_3$ | $C_5H_{11}$ sec | | |
| 2.47 | $NHCH_2$- 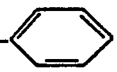 | $C_3H_7n$ | $C_2H_5$ | | Smp. 116–119° Beispiel 14 |
| 2.48 | $NHCH_2$- 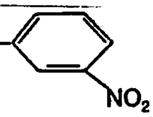 | $C_2H_4Cl$ | $C_2H_4F$ | | |
| 2.49 | $N(CH_3)CH_2$- 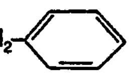 | $C_2H_4OC_2H_4$ | $CH_2CF_3$ | | |
| 2.50 | $NHC_2H_4OCH_3$ | $C_3H_7n$ | $C_2H_5$ | | |
| 2.51 | $NHC_2H_4SCH_3$ | $C_4H_9$ iso | $CH_3$ | | |
| 2.52 | Piperidino | $C_3H_7i$ | $CH_2\text{-}CH=CH_2$ | | |
| 2.53 | Morpholino | $C_6H_{13}$ | $C_2H_5$ | | |
| 2.54 | $OC_2H_5$ | $C_3H_7n$ | $C_2H_5$ | $Na^+$ | |
| 2.55 | $OC_2H_5$ | $C_3H_7n$ | $CH_2\text{-}CH=CH_2$ | $K^+$ | |
| 2.56 | $OC_4H_9$ iso | $C_3H_7n$ | $C_2H_5$ | ½ $Cu^{++}$ | |
| 2.57 | $OC_4H_9$ iso | $C_3H_7n$ | $CH_2\text{-}CH=CH_2$ | $NH_4^+$ | |
| 2.58 | $N(CH_3)_2$ | $C_3H_7n$ | $CH_2\text{-}CH=CH_2$ | $Na^+$ | |
| 2.59 | $NH_2$ | $C_2H_5$ | $CH_2\text{-}CCl=CH_2$ | $N(C_4H_9n)_4^+$ | |
| 2.60 | $OC_2H_5$ | $CH_3$ | $C_2H_5$ | | $n_D^{30}$ 1.5077 |
| 2.61 | $N(CH_3)OCH_3$ | $C_3H_7n$ | $C_2H_5$ | | $n_D^{25}$ 1.5122 |
| 2.62 | $N(CH_3)OCH_3$ | $C_3H_7n$ | $CH_2CH=CH_2$ | | |
| 2.63 | $N(CH_3)OCH_3$ | $C_3H_7n$ | $CH_2CH=CHCl$ | | Öl |
| 2.64 | $N(C_2H_5)_2$ | $C_3H_7n$ | $C_2H_5$ | | Wachs |
| 2.65 | $N(C_2H_5)_2$ | $C_3H_7n$ | $CH_2CH=CH_2$ | | |
| 2.66 | $N(CH_3)_2$ | Cyclopropyl | $C_2H_5$ | | |
| 2.67 | NH- 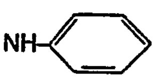 | Cyclopropyl | $CH_2CH=CH_2$ | | |
| 2.68 | $N(CH_3)OCH_3$ | $C_2H_5$ | $C_2H_5$ | | |
| 2.69 | $NHCH_2$- 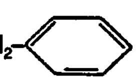 | $C_4H_9$ iso | $C_2H_5$ | | |
| 2.70 | $NHCH_2$- 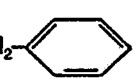 | Cyclopropyl | $CH_2CH=CH_2$ | | |
| 2.71 | NH- 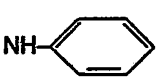 | $C_3H_7n$ | $C_2H_5$ | | |
| 2.72 | NH- 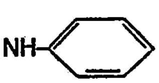 | $C_3H_7n$ | $CH_2CH=CH_2$ | | Wachs Beispiel 3 |
| 2.73 | NH- 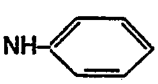 | $CH_3$ | $CH_2CH=CH_2$ | | |
| 2.74 | NH- 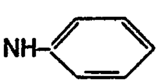 | $C_6H_{13}n$ | $C_2H_5$ | | |

| No. | A | R | $R_1$ | $M^{\oplus}$ | phys. Daten |
|---|---|---|---|---|---|
| 2.75 | 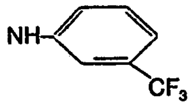 | $C_3H_7n$ | $C_2H_5$ | | |
| 2.76 | 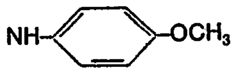 | $C_6H_{13}n$ | $C_2H_5$ | | |
| 2.77 | 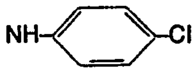 | $C_6H_{13}n$ | $CH_2CH=CH_2$ | | |
| 2.78 | 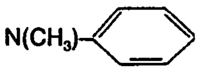 | $C_6H_{13}n$ | $CH_2CH=CH_2$ | | |
| 2.79 | Cyclopropylamino | $C_6H_{13}n$ | $C_2H_5$ | | |
| 2.80 | $NHC_6H_{13}n$ | $CH_3$ | $C_2H_5$ | | |
| 2.81 | 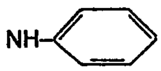 | $C_3H_7n$ | $CH_2CH=CHCl$ | | |
| 2.82 | $N(CH_3)_2$ | $C_3H_7n$ | $CH_2CH=CHCl$ | | |
| 2.83 | 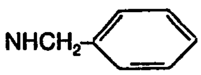 | $C_3H_7n$ | $CH_2CH=CHCl$ | | |
| 2.84 | $NHC_2H_4SCH_3$ | $C_3H_7n$ | $C_2H_5$ | | 61–67° |
| 2.85 | $NHC_2H_4SCH_3$ | $C_3H_7n$ | $CH_2CH=CH_2$ | | |
| 2.86 | $NHC_2H_4SCH_3$ | $C_3H_7n$ | $CH_2CH=CHCl$ | | |
| 2.87 | $N(CH_3)_2$ | Cyclopropyl | $CH_2CH=CH_2$ | | |
| 2.88 | $N(CH_3)_2$ | $C_3H_7n$ | $CH_2CH=CH_2$ | | Öl |
| 2.89 | $N(CH_3)_2$ | $C_3H_7n$ | $C_4H_9n$ | | Smp. 67–70° |
| 2.90 | $N(CH_3)_2$ | $C_3H_7n$ | $C_6H_{13}n$ | | Smp. 65–68° |
| 2.91 | $N(CH_3)_2$ | $C_2H_5$ | $C_2H_5$ | | |
| 2.92 | $N(CH_3)_2$ | $C_2H_5$ | $CH_2CH=CH_2$ | | Smp. 82–83° |
| 2.93 | $N(CH_3)_2$ | $C_6H_{13}n$ | $C_2H_5$ | | |
| 2.94 | $N(CH_3)_2$ | $C_5H_{11}n$ | $CH_2CH=CH_2$ | | Smp. 64–66° |
| 2.95 | 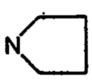 | $C_3H_7n$ | $C_2H_5$ | | Smp. 77–85° (Wachs) |
| 2.96 | 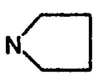 | $C_8H_7n$ | $CH_2–CH=CH_2$ | | |
| 2.97 | 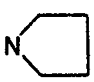 | $C_3H_7n$ | $CH_2–CH=CHCl$ | | |
| 2.98 | $NHCH_3$ | $C_3H_7n$ | $C_2H_5$ | | |
| 2.99 | $NHCH_3$ | $C_3H_7n$ | $CH_2CH=CH_2$ | | |
| 2.100 | $NHCH_3$ | $C_3H_7n$ | $CH_3$ | | Smp. 139–141° |
| 2.101 | 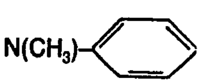 | $C_3H_7n$ | $CH_2CH=CH_2$ | | Smp. 52–56° |
| 2.102 | 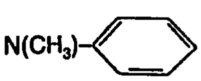 | $C_3H_7n$ | $C_2H_5$ | | Smp. 93–95° |

| No. | A | R | $R_1$ | $M^{\oplus}$ | phys. Daten |
|---|---|---|---|---|---|
| 2.103 | $N(CH_2CH{=}CH_2)_2$ | $C_3H_7n$ | $C_2H_5$ | | |
| 2.104 | $N(CH_2CH{=}CH_2)_2$ | $C_3H_7n$ | $CH_2CH{=}CH_2$ | | Öl |
| 2.105 | $N(CH_2CH{=}CH_2)_2$ | $C_3H_7n$ | $CH_2CH{=}CHCl$ | | Öl |
| 2.106 | $N(CH_2CH{=}CH_2)_2$ | $C_3H_7n$ | $CH_3$ | | |
| 2.107 | $NHCH_3$ | $C_3H_7n$ | $C_6H_{13}n$ | | |
| 2.108 | $NHCH_3$ | $C_3H_7n$ | $C_4H_9n$ | | |
| 2.109 | $NHCH_3$ | $C_5H_{11}n$ | $Ch_2CH{=}CH_2$ | | |
| 2.110 | $NHCH_3$ | $C_6H_{13}n$ | $C_4H_9n$ | | |
| 2.111 | $NHC_2H_4SCH_3$ | $C_2H_5$ | $CH_2CH{=}CH_2$ | | |
| 2.112 | $NHC_2H_4SC_3H_7i$ | $C_3H_7n$ | $CH_2CH{=}CH_2$ | | |
| 2.113 | $NHC_2H_4SC_3H_7i$ | $C_3H_7n$ | $C_4H_9n$ | | |
| 2.114 | $N(CH_3)C_2H_5$ | $C_3H_7n$ | $CH_2CH{=}CH_2$ | | |
| 2.115 | $N(CH_3)C_2H_5$ | $C_3H_7n$ | $C_4H_9n$ | | |
| 2.116 | $N(CH_3)C_2H_5$ | $C_3H_7n$ | $CH_2CH{=}CH_2Cl$ | | |
| 2.117 | $N(CH_3)_2$ | $C_5H_{11}n$ | $C_2H_5$ | | |

Beispiel 5:
Formulierungsbeispiele für Wirkstoffe der Formel I (% = Gewichtsprozent

| a) Spritzpulver | a) | b) | c) |
|---|---|---|---|
| Wirkstoff | 20% | 60% | 0,5% |
| Na-Ligninsulfonat | 5% | 5% | 5% |
| Na-Laurylsulfat | 3% | – | – |
| Na-Diisobutyl-naphthalinsulfonat | – | 6% | 6% |
| Octylphenolpoly-äthylenglykoläther (7–8 Mol AeO) | – | 2% | 2% |
| Hochdisperse Kiesel-säure | 5% | 27% | 27% |
| Kaolin | 67% | – | – |
| Natriumchlorid | – | – | 59,5% |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff | 10% | 1% |
| Octylphenolpolyäthylenglykoläther (4–5 Mol AeO) | 3% | 3% |
| Ca-Dodecylbenzolsulfonat | 3% | 3% |
| Ricinusölpolyglykoläther (36 Mol AeO) | 4% | 4% |
| Cyclohexanon | 30% | 10% |
| Xylolgemisch | 50% | 79% |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff | 0,1% | 1% |
| Talkum | 99,9% | – |
| Kaolin | – | 99% |

Man erhält anwendungsfertige Stäubemittel, indem der Wirkstoff mit dem Träger vermischt und auf einer geeigneten Mühle vermahlen wird.

| d) Extruder-Granulat | a) | b) |
|---|---|---|
| Wirkstoff | 10% | 1% |
| Na-Ligninsulfonat | 2% | 2% |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungsgranulat | |
|---|---|
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff | 40% | 5% |
| Äthylenglykol | 10% | 10% |

| f) Suspensions-Konzentrat | a) | b) |
|---|---|---|
| Nonylphenolpolyäthylenglykoläther (15 Mol AeO) | 6% | 1% |
| Na-Ligninsulfonat | 10% | 5% |
| Carboxymethylcellulose | 1% | 1% |
| 37%ige wässrige Formaldehyd-Lösung | 0,2% | 0,2% |
| Silikonöl in Form einer 75%igen wässrigen Emulsion | 0,8% | 0,8% |
| Wasser | 32% | 77% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| g) Salzlösung | |
|---|---|
| Wirkstoff | 5% |
| Isopropylamin | 1% |
| Octylphenolpolyäthylenglykoläther (78 Mol AeO) | 3% |
| Wasser | 91% |

Beispiel 6:
Herbizidwirkung vor dem Auflaufen der Pflanzen

Im Gewächshaus werden Pflanzensamen in Blumentöpfe von 11 cm Durchmesser gesät. Unmittelbar danach wird die Erdoberfläche mit einer wässrigen Emulsion der Wirkstoffe behandelt. Es wird eine Konzentration von 4 kg Wirkstoffmenge pro Hektar angewendet. Die Töpfe werden dann im Gewächshaus bei einer Temperatur von 22–25 °C und 50–70% relativer Luftfeuchtigkeit gehalten. Nach 3 Wochen wird der Versuch ausgewertet und die Wirkung auf die Versuchspflanzen nach dem folgenden Massstab bewertet:

1: Pflanze nicht gekeimt oder total abgestorben
2–3: sehr starke Wirkung
4–6: mittlere Wirkung
7–8: schwache Wirkung
9: keine Wirkung (wie unbehandelte Kontrolle).

Die Resultate sind wie folgt:

| Planze: | Avena sativa | Setaria italica | Sinapis alba | Stellaria media |
|---|---|---|---|---|
| Wirkstoff | | | | |
| 2.37 | 1 | 1 | 9 | 9 |
| 2.47 | 1 | 1 | 9 | 9 |
| 2.61 | 1 | 1 | 9 | 9 |
| 2.63 | 3 | 1 | 9 | 9 |
| 2.64 | 1 | 2 | 8 | 8 |

Beispiel 7:
Herbizide Wirkung bei Applikation der Wirkstoffe nach dem Auflaufen der Pflanzen

Verschiedene Kulturpflanzen und Unkräuter werden aus den Samen in Töpfen im Gewächshaus aufgezogen, bis sie das 4- bis 6-Blatt-Stadium erreicht haben. Dann werden die Pflanzen mit wässrigen Wirkstoffemulsionen (erhalten aus dem 25%igen Emulsionskonzentrat) in einer Dosierung von 4 kg/ha gespritzt. Die behandelten Pflanzen werden dann bei optimalen Bedingungen von Licht, regelmässigem Begiessen, 22–25 °C Temperatur und 50–70%iger relativer Luftfeuchtigkeit gehalten. Die Auswertung des Versuches erfolgt 15 Tage nach der Behandlung nach dem oben gegebenen Bewertungsschema. Die Resultate sind wie folgt:

| Pflanze: | Avena sativa | Setaria italica | Lolium perenne | Solanum lycopersicum | Sinapis alba | Stellaria media | Phaseolus vulgaris |
|---|---|---|---|---|---|---|---|
| Wirkstoff | | | | | | | |
| 2.37 | 1 | 2 | 2 | 9 | 8 | 8 | 7 |
| 2.47 | 2 | 4 | 2 | 4 | 6 | 8 | 9 |
| 2.61 | 1 | 2 | 2 | 7 | 7 | 8 | 9 |
| 2.63 | 1 | 1 | 2 | 8 | 5 | 8 | 5 |
| 2.64 | 1 | 1 | 2 | 4 | 7 | 8 | 9 |

Beispiel 8:
Wuchshemmung bei tropischen Bodenbedecker-Leguminosen (cover crops)

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70% relativer Luftfeuchtigkeit und 6000 lux Kunstlicht, pro Tag 14 Stunden, bei Temperaturen von 27° bei Tag und 21 °C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20% des Neuzuwachses bei unbehandelten Kontrollpflanzen), ohne dass dabei die Versuchspflanzen geschädigt wurden.

Beispiel 9:
Wuchsregulierung an Sojabohnen

In Kunststoffbehältern mit einem Erde-Torf-Sandgemisch im Verhältnis 6:3:1 werden Sojabohnen der Sorte «Hark» angesät und in eine Klimakammer gegeben. Durch optimale Temperaturwahl, Beleuchtung, Düngerzugabe und Bewässerung entwickeln sich die Pflanzen nach ca. 5 Wochen bis zum 5–6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der Schoten im Haupttrieb.

Beispiel 10:
Wuchshemmung bei Getreide

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60–90% der Kontrolle), sowie teilweise eine Zunahme der Stengeldurchmesser auf.

Beispiel 11:
Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerate und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet bis zu 100 g Aktivsubstanz pro Hektar. 21 Tage nach Applikation wird das Wachstum der Gräser beurteilt.

Die geprüften Verbindungen bewirken eine Reduktion des Neuzuwachses von um 10–30% im Vergleich zur unbehandelten Kontrolle.

**Patentansprüche für die Vertragsstaaten BE, LU, NL, SE**

1. Cyclohexandioncarbonsäurederivate der Formel I

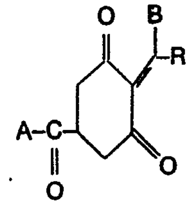

(I),

worin
A einen Rest $-OR_2$ oder $-NR_3R_4$,
B Hydroxyl, einen Rest $-NHOR_1$,
R $C_1$–$C_6$ Alkyl oder $C_3$–$C_6$ Cycloalkyl, unsubstituiert oder substituiert durch Halogen, $C_1$–$C_4$ Alkoxy oder $C_1$–$C_4$ Alkylthio,
$R_1$ $C_1$–$C_6$ Alkyl, $C_1$–$C_6$-Halogenalkyl, $C_3$–$C_6$ Alkenyl, $C_3$–$C_6$-Halogenalkenyl oder $C_3$–$C_6$ Alkinyl,
$R_2$, $R_3$ und $R_4$ unabhängig voneinander je Wasserstoff; $C_1$–$C_6$ Alkyl, $C_1$–$C_6$ Halogenalkyl, $C_2$–$C_{10}$ Alkoxyalkyl, $C_2$–$C_{10}$ Alkylthioalkyl; $C_3$–$C_6$ Alkenyl, unsubstituiert oder substituiert durch Halogen, $C_1$–$C_4$ Alkoxy oder $C_1$–$C_4$ Alkylthio; $C_3$–$C_6$ Alkinyl; Phenyl oder $C_1$–$C_6$ Aralkyl, wobei der Phenylkern unsubstituiert oder durch Halogen, $C_1$–$C_4$ Alkyl, $C_1$–$C_4$ Alkoxy, $C_1$–$C_4$ Halogenalkyl, Nitro oder Cyan substituiert ist,
$R_3$ und $R_4$ zusammen mit dem Stickstoffatom, an das sie gebunden sind, bilden auch einen 5–6 gliedrigen Heterocyclus, der im Ring noch ein Sauerstoff- oder Schwefelatom enthalten kann,
bedeuten, oder ein Metall- oder Ammoniumsalz eines solchen Cyclohexandioncarbonsäurederivats.

2. Cyclohexandioncarbonsäurederivate gemäss Anspruch 1 der Formel Ia

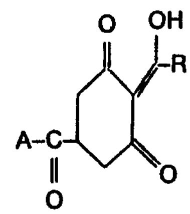

(Ia),

worin A und R die im Anspruch 1 gegebene Bedeutung haben oder ein Metall- oder Ammoniumsalz einer solchen Verbindung.

3. Cyclohexandioncarbonsäurederivate gemäss Anspruch 2, worin A einen Rest $-OR_2$ bedeutet und R und $R_2$ die im Anspruch 1 gegebene Bedeutung haben oder ein Metall- oder Ammoniumsalz einer solchen Verbindung.

4. Cyclohexandioncarbonsäurederivate gemäss Anspruch 2, worin A einen Rest $-NR_3R_4$ bedeutet und R, $R_3$ und $R_4$ die im Anspruch 1 gegebene Bedeutung haben oder ein Metall- oder Ammoniumsalz einer solchen Verbindung.

5. Cyclohexandioncarbonsäurederivate gemäss Anspruch 2, worin A die im Anspruch 1 gegebene Bedeutung hat und R einen $C_3$–$C_6$-Cycloalkylrest bedeutet oder ein Metall- oder Ammoniumsalz einer solchen Verbindung.

6. Cyclohexandioncarbonsäurederivate gemäss Anspruch 1 der Formel Ib

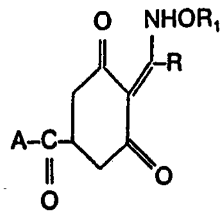

(Ib)

worin A, R und $R_1$ die im Anspruch 1 gegebene Bedeutung haben, oder ein Metall- oder quaternäres Ammoniumsalz einer solchen Verbindung.

7. Cyclohexandioncarbonsäurederivate gemäss Anspruch 6, worin A einen Rest $OR_2$ bedeutet und R, $R_1$ und $R_2$ die im Anspruch 1 gegebene Bedeutung haben oder ein Metall- oder quaternäres Ammoniumsalz einer solchen Verbindung.

8. Cyclohexandioncarbonsäurederivate gemäss Anspruch 6, worin A einen Rest $-NR_3R_4$ bedeutet und R, $R_1$, $R_3$ und $R_4$ die im Anspruch 1 gegebene Bedeutung haben oder ein Metall- oder quaternäres Ammoniumsalz einer solchen Verbindung.

9. 4-Butyryl-3,5-cyclohexandioncarbonsäure-äthylester
gemäss Anspruch 2.

10. 4-Buturyl-3,5-cyclohexandioncarbonsäure-isobutylester
gemäss Anspruch 2.

11. 4-(Cyclopropyl-hydroxymethyliden)-3,5-cyclohexandioncarbonsäureäthylester
gemäss Anspruch 2.

12. 4-(1-Äthoxyaminobutyliden)-3,5-cyclohexandioncarbonsäuredimethylamid
gemäss Anspruch 6.

13. 4-(1-Allyloxyaminobutyliden)-3,5-cyclohexandioncarbonsäurediäthylamid
gemäss Anspruch 6.

14. 4-(1-Äthoxyaminobutyliden)-3,5-cyclohexandioncarbonsäurebenzylamid
gemäss Anspruch 6.

15. 4-(1-Allyloxyaminobutyliden)-3,5-cyclohexandioncarbonsäure-isobutylester
gemäss Anspruch 6.

16. Verfahren zur Herstellung der Cyclohexandioncarbonsäurederivate der Formel I, gemäss Anspruch 1, dadurch gekennzeichnet, dass man ein 3,5-Cyclohexandioncarbonsäurederivat der Formel II

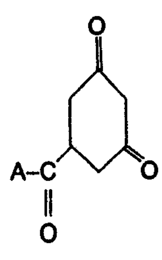

(II),

worin A einen wie in Anspruch 1 definierten Ester- oder Amidrest darstellt, in einem inerten organischen Lösungsmittel, in Gegenwart einer Base als säurebindendes Mittel, mit einem Säurehalogenid der Formel III umsetzt

$$Hal-COR \qquad (III),$$

worin R die in Anspruch 1 gegebene Bedeutung hat, das erhaltene Produkt isoliert und gegebenenfalls weiter in einem inerten organischen Lösungsmittel bei Siedetemperatur unter wasserabspaltenden Bedingungen mit einem Hydroxylamin der Formel IV umsetzt

$$HONHR_1 \qquad (IV),$$

worin $R_1$ die oben gegebene Bedeutung hat und das erhaltene Produkt isoliert.

17. Ein herbizides und den Pflanzenwuchs regulierendes Mittel, dadurch gekennzeichnet, dass es neben Träger- und/oder anderen Zuschlagstoffen als Wirkstoff mindestens ein Cyclohexandioncarbonsäurederivat der Formel I, gemäss Anspruch 1, enthält.

18. Die Verwendung der Cyclohexandioncarbonsäurederivate der Formel I, gemäss Anspruch 1, oder sie enthaltender Mittel zur Bekämpfung unerwünschten Pflanzenwachstums.

19. Die Verwendung der Cyclohexandioncarbonsäurederivate der Formel I, gemäss Anspruch 1, oder sie enthaltender Mittel zur Regulierung des Pflanzenwachstums.

20. Die Verwendung der Cyclohexandioncarbonsäurederivate der Formel I, gemäss Anspruch 1, oder sie enthaltender Mittel zur Wuchshemmung von Gräsern.

21. Verfahren zur selektiven pre- oder postemergenten Bekämpfung von Unkräutern in Nutzpflanzenkulturen, dadurch gekennzeichnet, dass man die Nutzpflanzen oder deren Anbaufläche mit einer wirksamen Menge eines Cyclohexandioncarbonsäurederivates der Formel I, gemäss Anspruch 1, oder eines ein solches Derivate enthaltenden Mittels behandelt.

22. Verfahren zur selektiven pre- und postemergenten Bekämpfung von Ungräsern in Nutzpflanzenkulturen, dadurch gekennzeichnet, dass man die Nutzpflanzen oder deren Anbaufläche mit einer wirksamen Menge eines Cyclohexandioncarbonsäurederivates der Formel I, gemäss Anspruch 1, oder eines ein solches Derivat enthaltenden Mittels behandelt.

23. Verfahren zur Regulierung des Pflanzenwuchses, dadurch gekennzeichnet, dass man Pflanzen, Pflanzenteile oder Samen mit einer wirksamen Menge eines Cyclohexandioncarbonsäureesters der Formel I, gemäss Anspruch 1, oder eines ein solches Derivat enthaltenden Mittels behandelt.

24. Die Verwendung der Cyclohexandioncarbonsäurederivate der Formel I, oder sie enthaltender Mittel zur Unterdrückung des Pflanzenwachstums über das 2-Blattstadium hinaus, dadurch gekennzeichnet, dass die Wirkstoffe preemergent angewendet werden.

25. Die Verwendung gemäss Anspruch 22 in Zuckerrohr-, Getreide-, Mais-, Soja-, Reis- und Baumwollkulturen.

26. Die Verwendung der Cyclohexandioncarbonsäurederivate der Formel I, gemäss Anspruch 1, oder sie enthaltender Mittel zur Wuchsregulierung von Kulturpflanzen zum Zwecke einer Ertragssteigerung.

27. Die Verwendung gemäss Anspruch 26 in Sojakulturen.

28. Die Verwendung gemäss Anspruch 24 bei Bodenbedecker-Leguminosen.

**Patentansprüche für die Vertragsstaaten CH, DE, FR, GB, IT, LI**

1. Cyclohexandioncarbonsäurederivate der Formel I

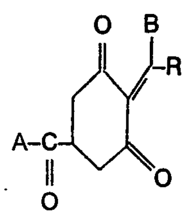

(I),

worin

A einen Rest $-OR_2$ oder $-NR_3R_4$,

B Hydroxyl, einen Rest $-NHOR_1$,

R $C_1$–$C_6$ Alkyl oder $C_3$–$C_6$ Cycloalkyl, unsubstituiert oder substituiert durch Halogen, $C_1$–$C_4$ Alkoxy oder $C_1$–$C_4$ Alkylthio,

$R_1$ $C_1$–$C_6$ Alkyl, $C_1$–$C_6$-Halogenalkyl, $C_3$–$C_6$ Alkenyl, $C_3$–$C_6$-Halogenalkenyl oder $C_3$–$C_6$ Alkinyl,

$R_2$, $R_3$ und $R_4$ unabhängig voneinander je Wasserstoff; $C_1$–$C_6$ Alkyl, $C_1$–$C_6$ Halogenalkyl, $C_2$–$C_{10}$ Alkoxyalkyl, $C_2$–$C_{10}$ Alkylthioalkyl; $C_3$–$C_6$ Alkenyl, unsubstituiert oder substituiert durch Halogen, $C_1$–$C_4$ Alkoxy oder $C_1$–$C_4$ Alkylthio; $C_3$–$C_6$ Alkinyl; Phenyl oder $C_1$–$C_6$ Aralkyl, wobei der Phenylkern unsubstituiert oder durch Halogen, $C_1$–$C_4$ Alkyl, $C_1$–$C_4$ Alkoxy, $C_1$–$C_4$ Halogenalkyl, Nitro oder Cyan substituiert ist,

$R_3$ und $R_4$ zusammen mit dem Stickstoffatom, an das sie gebunden sind, bilden auch einen 5–6 gliedrigen Heterocyclus, der im Ring noch ein Sauerstoff- oder Schwefelatom enthalten kann,

bedeuten, oder ein Metall- oder Ammoniumsalz eines solchen Cyclohexandioncarbonsäurederivats, wobei im Falle von Salzen R nicht $C_1$–$C_6$-Alkyl bedeutet, wenn $R_2$ Wasserstoff oder $C_1$–$C_6$-Alkyl ist.

2. Cyclohexandioncarbonsäurederivate gemäss Anspruch 1 der Formel Ia

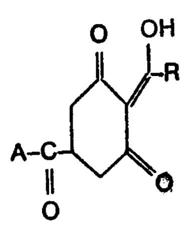

(Ia),

worin A und R die im Anspruch 1 gegebene Bedeutung haben oder ein Metall- oder Ammoniumsalz einer solchen Verbindung.

3. Cyclohexandioncarbonsäurederivate gemäss Anspruch 2, worin A ein Rest $-OR_2$ bedeutet und R und $R_2$ die im Anspruch 1 gegebene Bedeutung haben oder ein Metall- oder Ammoniumsalz einer solchen Verbindung.

4. Cyclohexandioncarbonsäurederivate gemäss Anspruch 2, worin A einen Rest $-NR_3R_4$ bedeutet und R, $R_3$ und $R_4$ die im Anspruch 1 gegebene Bedeutung haben oder ein Metall- oder Ammoniumsalz einer solchen Verbindung.

5. Cyclohexandioncarbonsäurederivate gemäss Anspruch 2, worin A die im Anspruch 1 gegebene Bedeutung hat und R einen $C_3$–$C_6$-Cycloalkylrest bedeutet oder ein Metall- oder Ammoniumsalz einer solchen Verbindung.

6. Cyclohexandioncarbonsäurederivate gemäss Anspruch 1 der Formel Ib

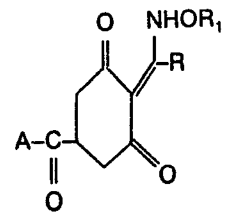

(Ib)

worin A, R und $R_1$ die im Anspruch 1 gegebene Bedeutung haben, oder ein Metall- oder quaternäres Ammoniumsalz einer solchen Verbindung.

7. Cyclohexandioncarbonsäurederivate gemäss Anspruch 6, worin A einen Rest $OR_2$ bedeutet und R, $R_1$ und $R_2$ die im Anspruch 1 gegebene Bedeutung haben oder ein Metall- oder quaternäres Ammoniumsalz einer solchen Verbindung.

8. Cyclohexandioncarbonsäurederivate gemäss Anspruch 6, worin A einen Rest $-NR_3R_4$ bedeutet und R, $R_1$, $R_3$ und $R_4$ die im Anspruch 1 gegebene Bedeutung haben oder ein Metall- oder quaternäres Ammoniumsalz einer solchen Verbindung.

9. 4-Butyryl-3,5-cyclohexandioncarbonsäureäthylester
gemäss Anspruch 2.

10. 4-Buturyl-3,5-cyclohexandioncarbonsäureisobutylester
gemäss Anspruch 2.

11. 4-(Cyclopropyl-hydroxymethyliden)-3,5-cyclohexandioncarbonsäureäthylester
gemäss Anspruch 2.

12. 4-(1-Äthoxyaminobutyliden)-3,5-cyclohexandioncarbonsäuredimethylamid
gemäss Anspruch 6.

13. 4-(1-Allyloxyaminobutyliden)-3,5-cyclohexandioncarbonsäurediäthylamid
gemäss Anspruch 6.

14. 4-(1-Äthoxyaminobutyliden)-3,5-cyclohexandioncarbonsäurebenzylamid
gemäss Anspruch 6.

15. 4-(1-Allyloxyaminobutyliden)-3,5-cyclohexandioncarbonsäure-isobutylester
gemäss Anspruch 6.

16. Verfahren zur Herstellung der Cyclohexandioncarbonsäurederivate der Formel I, gemäss Anspruch 1, dadurch gekennzeichnet, dass man ein 3,5-Cyclohexandioncarbonsäurederivat der Formel II

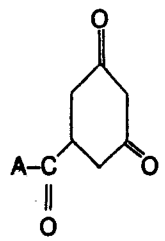

(II),

worin A einen wie in Anspruch 1 definierten Ester- oder Amidrest darstellt, in einem inerten organischen Lösungsmittel, in Gegenwart einer Base als säurebindendes Mittel, mit einem Säurehalogenid der Formel III umsetzt

Hal–COR (III),

worin R die in Anspruch 1 gegebene Bedeutung hat, das erhaltene Produkt isoliert und gegebenenfalls weiter in einem inerten organischen Lösungsmittel bei Siedetemperatur unter wasserabspaltenden Bedingungen mit einem Hydroxylamin der Formel IV umsetzt

$HONHR_1$ (IV),

worin $R_1$ die oben gegebene Bedeutung hat und das erhaltene Produkt isoliert.

17. Ein herbizides und den Pflanzenwuchs regulierendes Mittel, dadurch gekennzeichnet, dass es neben Träger- und/oder anderen Zuschlagstoffen als Wirkstoff mindestens ein Cyclohexandioncarbonsäurederivat der Formel I, gemäss Anspruch 1, enthält.

18. Die Verwendung der Cyclohexandioncarbonsäurederivate der Formel I, gemäss Anspruch 1, oder sie enthaltender Mittel zur Bekämpfung unerwünschten Pflanzenwachstums.

19. Die Verwendung der Cyclohexandioncarbonsäurederivate der Formel I, gemäss Anspruch 1, oder sie enthaltender Mittel zur Regulierung des Pflanzenwachstums.

20. Die Verwendung der Cyclohexandioncarbonsäurederivate der Formel I, gemäss Anspruch 1, oder sie enthaltender Mittel zur Wuchshemmung von Gräsern.

21. Verfahren zur selektiven pre- oder postemergenten Bekämpfung von Unkräutern in Nutzpflanzenkulturen, dadurch gekennzeichnet, dass man die Nutzpflanzen oder deren Anbaufläche mit einer wirksamen Menge eines Cyclohexandioncarbonsäurederivates der Formel I, gemäss Anspruch 1, oder eines ein solches Derivate enthaltenden Mittels behandelt.

22. Verfahren zur selektiven pre- und postemergenten Bekämpfung von Ungräsern in Nutzpflanzenkulturen, dadurch gekennzeichnet, dass man die Nutzpflanzen oder deren Anbaufläche mit einer wirksamen Menge eines Cyclohexandioncarbonsäurederivates der Formel I, gemäss Anspruch 1, oder eines ein solches Derivat enthaltenden Mittels behandelt.

23. Verfahren zur Regulierung des Pflanzenwuchses, dadurch gekennzeichnet, dass man Pflanzen, Pflanzenteile oder Samen mit einer wirksamen Menge eines Cyclohexandioncarbonsäureesters der Formel I, gemäss Anspruch 1, oder eines ein solches Derivat enthaltenden Mittels behandelt.

24. Die Verwendung der Cyclohexandioncarbonsäurederivate der Formel I, oder sie enthaltender Mittel zur Unterdrückung des Pflanzenwachstums über das 2-Blattstadium hinaus, dadurch gekennzeichnet, dass die Wirkstoffe preemergent angewendet werden.

25. Die Verwendung gemäss Anspruch 22 in Zuckerrohr-, Getreide-, Mais-, Soja-, Reis- und Baumwollkulturen.

26. Die Verwendung der Cyclohexandioncarbonsäurederivate der Formel I, gemäss Anspruch 1, oder sie enthaltender Mittel zur Wuchsregulierung von Kulturpflanzen zum Zwecke einer Ertragssteigerung.

27. Die Verwendung gemäss Anspruch 26 in Sojakulturen.

28. Die Verwendung gemäss Anspruch 24 bei Bodenbedecker-Leguminosen.

**Patentansprüche für den Vertragsstaat AT**

1. Herbizides und pflanzenwuchsregulierendes Mittel, dadurch gekennzeichnet, dass es neben Träger- und/oder anderen Zuschlagstoffen mindestens ein Cyclohexandioncarbonsäurederivat der Formel I

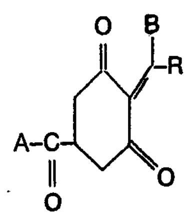

(I),

worin

A einen Rest $-OR_2$ oder $-NR_3R_4$,

B Hydroxyl oder einen Rest $-NHOR_1$,

R $C_1$–$C_6$ Alkyl oder $C_3$–$C_6$ Cycloalkyl, unsubstituiert oder substituiert durch Halogen, $C_1$–$C_4$ Alkoxy oder $C_1$–$C_4$ Alkylthio,

$R_1$ $C_1$–$C_6$ Alkyl, $C_1$–$C_6$-Halogenalkyl, $C_3$–$C_6$ Alkenyl, $C_3$–$C_6$-Halogenalkenyl oder $C_3$–$C_6$ Alkinyl,

$R_2$, $R_3$ und $R_4$ unabhängig voneinander je Wasserstoff; $C_1$–$C_6$ Alkyl, $C_1$–$C_6$ Halogenalkyl, $C_2$–$C_{10}$ Alkoxyalkyl, $C_2$–$C_{10}$ Alkylthioalkyl; $C_3$–$C_6$ Alkenyl, unsubstituiert oder substituiert durch Halogen, $C_1$–$C_4$ Alkoxy oder $C_1$–$C_4$ Alkylthio; $C_3$–$C_6$ Alkinyl; Phenyl oder $C_1$–$C_6$ Aralkyl, wobei der Phenylkern unsubstituiert oder durch Halogen, $C_1$–$C_4$ Alkyl, $C_1$–$C_4$ Alkoxy, $C_1$–$C_4$ Halogenalkyl, Nitro oder Cyan substituiert ist,

$R_3$ und $R_4$ zusammen mit dem Stickstoffatom, an das sie gebunden sind, bilden auch einen 5–6 gliedrigen Heterocyclus, der im Ring noch ein Sauerstoff- oder Schwefelatom enthalten kann, bedeuten, oder ein Metall- oder Ammoniumsalz eines solchen Cyclohexandioncarbonsäurederivats enthält.

2. Herbizides und pflanzenwuchsregulierendes Mittel gemäss Anspruch 1, dadurch gekennzeichnet, dass es ein Cyclohexandioncarbonsäurederivat der Formel Ia

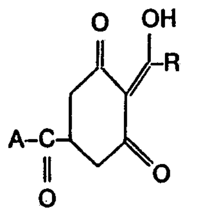

(Ia),

worin A und R die im Anspruch 1 gegebene Bedeutung haben oder ein Metall- oder Ammoniumsalz einer solchen Verbindung enthält.

3. Herbizides und pflanzenwuchsregulierendes Mittel gemäss Anspruch 2, dadurch gekennzeichnet, dass es ein Cyclohexandioncarbonsäurederivat der Formel Ia, worin A einen Rest $-OR_2$ bedeutet und R und $R_2$ die im Anspruch 1 gegebene Bedeutung haben oder ein Metall- oder Ammoniumsalz einer solchen Verbindung enthält.

4. Herbizides und pflanzenwuchsregulierendes Mittel gemäss Anspruch 2, dadurch gekennzeichnet, dass es ein Cyclohexandioncarbonsäurederivat der Formel Ia, worin A einen Rest $-NR_3R_4$ bedeutet und R, $R_3$ und $R_4$ die im Anspruch 1 gegebene Bedeutung haben oder ein Metall- oder Ammoniumsalz einer solchen Verbindung enthält.

5. Herbizides und pflanzenwuchsregulierendes Mittel gemäss Anspruch 2, dadurch gekennzeichnet, dass es ein Cyclohexandioncarbonsäurederivat der Formel Ia, worin A die im Anspruch 1 gegebene Bedeutung hat und R einen $C_3$–$C_6$-Cycloalkylrest bedeutet oder ein Metall- oder Ammoniumsalz einer solchen Verbindung enthält.

6. Herbizides und pflanzenwuchsregulierendes Mittel gemäss Anspruch 1, dadurch gekennzeichnet, dass es ein Cyclohexandioncarbonsäurederivat der Formel Ib

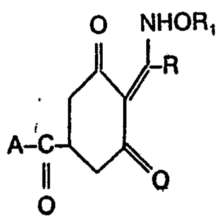

(Ib)

worin A, R und $R_1$ die im Anspruch 1 gegebene Bedeutung haben, oder ein Metall- oder quaternäres Ammoniumsalz einer solchen Verbindung enthält.

7. Herbizides und pflanzenwuchsregulierendes Mittel gemäss Anspruch 6, dadurch gekennzeichnet, dass es ein Cyclohexandioncarbonsäurederivat der Formel Ib, worin A einen Rest $OR_2$ bedeutet und R, $R_1$ und $R_2$ die im Anspruch 1 gegebene Bedeutung haben oder ein Metall- oder quaternäres Ammoniumsalz einer solchen Verbindung enthält.

8. Herbizides und pflanzenwuchsregulierendes Mittel gemäss Anspruch 6, dadurch gekennzeichnet, dass es ein Cyclohexandioncarbonsäurederivat der Formel Ib, worin A einen Rest $-NR_3R_4$ bedeutet und R, $R_1$, $R_3$ und $R_4$ die im Anspruch 1 gegebene Bedeutung haben oder ein Metall- oder ein quaternäres Ammoniumsalz einer solchen Verbindung enthält.

9. Herbizides und pflanzenwuchsregulierendes Mittel gemäss Anspruch 2, dadurch gekennzeichnet, dass es 4-Butyryl-3,5-cyclohexandioncarbonsäureäthylester enthält.

10. Herbizides und pflanzenwuchsregulierendes Mittel gemäss Anspruch 2, dadurch gekennzeichnet, dass es 4-Buturyl-3,5-cyclohexandioncarbonsäureisobutylester enthält.

11. Herbizides und pflanzenwuchsregulierendes Mittel gemäss Anspruch 2, dadurch gekennzeichnet, dass es 4-(Cyclopropyl-hydroxymethyliden)-3,5-cyclohexandioncarbonsäureäthylester enthält.

12. Herbizides und pflanzenwuchsregulierendes Mittel gemäss Anspruch 6, dadurch gekennzeichnet, dass es 4-(1-Äthoxyaminobutyliden)-3,5-cyclohexandioncarbonsäuredimethylamid enthält.

13. Herbizides und pflanzenwuchsregulierendes Mittel gemäss Anspruch 6, dadurch gekennzeichnet, dass es 4-(Allyloxyaminobutyliden)-3,5-cyclohexandioncarbonsäurediäthylamid enthält.

14. Herbizides und pflanzenwuchsregulierendes Mittel gemäss Anspruch 6, dadurch gekennzeichnet, dass es 4-(1-Äthoxyaminobutyliden)-3,5-cyclohexandioncarbonsäurebenzylamid enthält.

15. Herbizides und pflanzenwuchsregulierendes Mittel gemäss Anspruch 6, dadurch gekennzeichnet, dass es 4-(1-Allyloxyaminobutyliden)-3,5-cyclohexandioncarbonsäureisobutylester enthält.

16. Verfahren zur Herstellung der Cyclohexandioncarbonsäurederivate der Formel I, gemäss Anspruch 1, dadurch gekennzeichnet, dass man ein 3,5-Cyclohexandioncarbonsäurederivat der Formel II

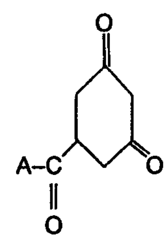

(II),

worin A einen wie in Anspruch 1 definierten Ester- oder Amidrest darstellt, in einem inerten organischen Lösungsmittel, in Gegenwart einer Base als säurebindendes Mittel, mit einem Säurehalogenid der Formel III umsetzt

Hal–COR (III),

worin R die in Anspruch 1 gegebene Bedeutung hat, das erhaltene Produkt isoliert und gegebenenfalls weiter in einem inerten organischen Lösungsmittel bei Siedetemperatur unter wasserabspaltenden Bedingungen mit einem Hydroxylamin der Formel IV umsetzt

$HONHR_1$ (IV),

worin $R_1$ die oben gegebene Bedeutung hat und das erhaltene Produkt gewünschtenfalls in ein Metall- oder Ammoniumsalz überführt.

17. Die Verwendung der Cyclohexandioncarbonsäurederivate der Formel I, gemäss Anspruch 1, oder sie enthaltender Mittel zur Bekämpfung unerwünschten Pflanzenwachstums.

18. Die Verwendung der Cyclohexandioncarbonsäurederivate der Formel I, gemäss Anspruch 1, oder sie enthaltender Mittel zur Regulierung des Pflanzenwachstums.

19. Die Verwendung der Cyclohexandioncarbonsäurederivate der Formel I, gemäss Anspruch 1, oder sie enthaltender Mittel zur Wuchshemmung von Gräsern.

20. Verfahren zur selektiven pre- oder postemergenten Bekämpfung von Unkräutern in Nutzpflanzenkulturen, dadurch gekennzeichnet, dass man die Nutzpflanzen oder deren Anbaufläche mit einer wirksamen Menge eines Cyclohexandioncarbonsäurederivates der Formel I, gemäss Anspruch 1, oder eines ein solches Derivate enthaltenden Mittels behandelt.

21. Verfahren zur selektiven pre- und postemergenten Bekämpfung von Ungräsern in Nutzpflanzenkulturen, dadurch gekennzeichnet, dass man die Nutzpflanzen oder deren Anbaufläche mit einer wirksamen Menge eines Cyclohexandioncarbonsäurederivates der Formel I, gemäss Anspruch 1, oder eines ein solches Derivat enthaltenden Mittels behandelt.

22. Verfahren zur Regulierung des Pflanzenwuchses, dadurch gekennzeichnet, dass man Pflanzen, Pflanzenteile oder Samen mit einer wirksamen Menge eines Cyclohexandioncarbonsäureesters der Formel I, gemäss Anspruch 1, oder eines ein solches Derivat enthaltenden Mittels behandelt.

23. Die Verwendung der Cyclohexandioncarbonsäurederivate der Formel I, oder sie enthaltender Mittel zur Unterdrückung des Pflanzenwachstums über das 2-Blattstadium hinaus, dadurch gekennzeichnet, dass die Wirkstoffe preemergent angewendet werden.

24. Die Verwendung gemäss Anspruch 21 in Zuckerrohr-, Getreide-, Mais-, Soja-, Reis- und Baumwollkulturen.

25. Die Verwendung der Cyclohexandioncarbonsäurederivate der Formel I, gemäss Anspruch 1, oder sie enthaltender Mittel zur Wuchsregulierung von Kulturpflanzen zum Zwecke einer Ertragssteigerung.

26. Die Verwendung gemäss Anspruch 25 in Sojakulturen.

27. Die Verwendung gemäss Anspruch 23 bei Bodenbedecker-Leguminosen.

**Claims for the Contracting States: BE, LU, NL, SE**

1. Cyclohexanedionecarboxylic acid derivatives of the formula I

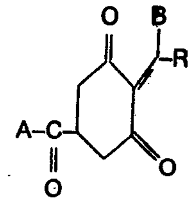

(I)

wherein

A is an $-OR_2$ or $-NR_3R_4$ radical,

B is hydroxyl or an $-NHOR_1$ radical,

R is $C_1$–$C_6$alkyl or $C_3$–$C_6$cycloalkyl, each unsubstituted or substituted by halogen, $C_1$–$C_4$alkoxy or $C_1$–$C_4$alkylthio,

$R_1$ is $C_1$–$C_6$alkyl, $C_1$–$C_6$haloalkyl, $C_3$–$C_6$alkenyl, $C_3$–$C_6$haloalkenyl or $C_3$–$C_6$alkynyl,

$R_2$, $R_3$ and $R_4$ are each independently hydrogen, $C_1$–$C_6$alkyl, $C_1$–$C_6$haloalkyl, $C_2$–$C_{10}$alkoxyalkyl, $C_2$–$C_{10}$alkylthioalkyl; $C_3$–$C_6$alkenyl which is unsubstituted or substituted by halogen, $C_1$–$C_4$alkoxy or $C_1$–$C_4$alkylthio; $C_3$–$C_6$alkynyl; phenyl or $C_1$–$C_6$aralkyl, wherein the phenyl nucleus is unsubstituted or substituted by halogen, $C_1$–$C_4$alkyl, $C_1$–$C_4$alkoxy, $C_1$–$C_4$haloalkyl, nitro or cyano, and

$R_3$ and $R_4$, together with the nitrogen atom to which they are bonded, also form a 5- or 6-membered heterocyclic ring system which may also contain an oxygen or sulfur atom in the ring,

or a metal or ammonium salt of such a cyclohexanedionecarboxylic acid derivative.

2. Cyclohexanedionecarboxylic acid derivatives according to claim 1 of the formula Ia

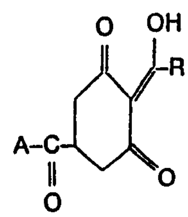

(Ia)

wherein A and R are as defined in claim 1, or a metal or ammonium salt thereof.

3. Cyclohexanedionecarboxylic acid derivatives according to claim 2, wherein A is an $-OR_2$ radical and R and $R_2$ are as defined in claim 1, or a metal or ammonium salt thereof.

4. Cyclohexanedionecarboxylic acid derivatives according to claim 2, wherein A is an $-NR_3R_4$ radical and R, $R_3$ and $R_4$ are as defined in claim 1, or a metal or ammonium salt thereof.

5. Cyclohexanedionecarboxylic acid derivatives according to claim 2, wherein A is as defined in claim 1 and R is a $C_3$–$C_6$cycloalkyl radical, or a metal or ammonium salt thereof.

6. Cyclohexanedionecarboxylic acid derivatives according to claim 1 of the formula Ib

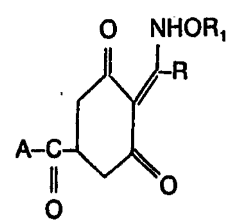

(Ib)

wherein A, R and $R_1$ are as defined in claim 1, or a metal or quaternary ammonium salt thereof.

7. Cyclohexanedionecarboxylic acid derivatives according to claim 6, wherein A is an $-OR_2$ radical and R, $R_1$ and $R_2$ are as defined in claim 1, or a metal or quaternary ammonium salt thereof.

8. Cyclohexanedionecarboxylic acid derivatives according to claim 6, wherein A is an $-NR_3R_4$ radical and R, $R_1$, $R_3$ and $R_4$ are as defined in claim 1, or a metal or quaternary ammonium salt thereof.

9. Ethyl 4-butyryl-3,5-cyclohexanedionecarboxylate according to claim 2.

10. Isobutyl 4-butyryl-3,5-cyclohexanedionecarboxylate according to claim 2.

11. Ethyl 4-(cyclopropylhydroxymethylidene)-3,5-cyclohexanedionecarboxylate according to claim 2.

12. Dimethyl 4-(1-ethoxyaminobutylidene)-3,5-cyclohexanedionecarboxamide according to claim 6.

13. Diethyl 4-(1-allyloxyaminobutylidene)-3,5-cyclohexanedionecarboxamide according to claim 6.

14. Benzyl 4-(1-ethoxyaminobutylidene)-3,5-cyclohexanedionecarboxamide according to claim 6.

15. Isobutyl 4-(1-allyloxyaminobutylidene)-3,5-cyclohexanedionecarboxylate according to claim 6.

16. A process for the preparation of cyclohexanedionecarboxylic acid derivatives of the formula I according to claim 1, which comprises reacting a 3,5-cyclohexanedionecarboxylic acid derivative of the formula II

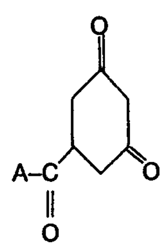

(II)

wherein A is an ester or amide radical as defined in claim 1, with an acid halide of the formula III

Hal–COR (III)

wherein R is as defined in claim 1, in an inert organic solvent and in the presence of a base as acid acceptor, isolating the product so obtained and, if desired, reacting it further with a hydroxylamine of the formula IV

$HONHR_1$ (IV)

wherein $R_1$ is as defined above, in an inert organic solvent at boiling temperature under condensation conditions, and isolating the resultant product.

17. A herbicidal and plant-growth-regulating composition, which contains, as active ingredient, at least one cyclohexanedionecarboxylic acid derivative of the formula I according to claim 1, together with carriers and/or other adjuvants.

18. The use of the cyclohexanedionecarboxylic acid derivatives of the formula I according to claim 1, or of compositions containing such compounds, for controlling undesirable plant growth.

19. The use of the cyclohexanedionecarboxylic acid derivatives of the formula I according to claim 1, or of compositions containing such compounds, for regulating plant growth.

20. The use of the cyclohexanedionecarboxylic acid derivatives of the formula I according to claim 1, or of compositions containing such compounds, for inhibiting the growth of grasses.

21. A method of selectively controlling weeds pre- or postemergence in crops of useful plants, which comprises treating the useful plants or the locus thereof with an effective amount of a cyclohexanedionecarboxylic acid derivative of the formula I according to claim 1, or of a composition containing such a derivative.

22. A method of selectively controlling weed grasses pre- and postemergence in crops of useful plants, which comprises treating the useful plants or the locus thereof with an effective amount of a cyclohexanedionecarboxylic acid derivative of the formula I according to claim 1, or of a composition containing such a derivative.

23. A method of regulating plant growth, which comprises treating plants, parts of plants or seeds with an effective amount of a cyclohexanedionecarboxylic acid ester of the formula I according to claim 1, or of a composition containing such a derivative.

24. The use of the cyclohexanedionecarboxylic acid derivatives of the formula I, or of compositions containing such compounds, for inhibiting plant growth beyond the 2-leaf stage, wherein the active ingredients are applied pre-emergence.

25. The use according to claim 22 in crops of sugar cane, cereals, maize, soybeans, rice and cotton.

26. The use of the cyclohexanedionecarboxylic acid derivatives of the formula I according to claim 1, or of compositions containing such compounds, for regulating the growth of cultivated plants for the purpose of increasing yield.

27. The use according to claim 26 in crops of soybeans.

28. The use according to claim 24 in leguminous cover crops.

**Claims for the Contracting States: CH, DE, FR, GB, IT, LI**

1. Cyclohexanedionecarboxylic acid derivatives of the formula I

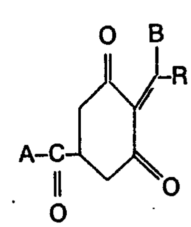

(I)

wherein

A is an $-OR_2$ or $-NR_3R_4$ radical,

B is hydroxyl or an $-NHOR_1$ radical,

R is $C_1$–$C_6$alkyl or $C_3$–$C_6$cycloalkyl, each unsubstituted or substituted by halogen, $C_1$–$C_4$alkoxy or $C_1$–$C_4$alkylthio,

$R_1$ is $C_1$–$C_6$alkyl, $C_1$–$C_6$haloalkyl, $C_3$–$C_6$alkenyl, $C_3$–$C_6$haloalkenyl or $C_3$–$C_6$alkynyl,

$R_2$, $R_3$ and $R_4$ are each independently hydrogen, $C_1$–$C_6$alkyl, $C_1$–$C_6$haloalkyl, $C_2$–$C_{10}$alkoxyalkyl, $C_2$–$C_{10}$alkylthioalkyl; $C_3$–$C_6$alkenyl which is unsubstituted or substituted by halogen, $C_1$–$C_4$alkoxy or $C_1$–$C_4$alkylthio; $C_3$–$C_6$alkynyl; phenyl or $C_1$–$C_6$aralkyl, wherein the phenyl nucleus is unsubstituted or substituted by halogen, $C_1$–$C_4$alkyl, $C_1$–$C_4$alkoxy, $C_1$–$C_4$haloalkyl, nitro or cyano, and

$R_3$ and $R_4$, together with the nitrogen atom to which they are bonded, also form a 5- or 6-membered heterocyclic ring system which may also contain an oxygen or sulfur atom in the ring,

or a metal or ammonium salt of such a cyclohexanedionecarboxylic acid derivative, wherein, in the case of salts, R is not $C_1$–$C_6$alkyl when $R_2$ is hydrogen or $C_1$–$C_6$alkyl.

2. Cyclohexanedionecarboxylic acid derivatives according to claim 1 of the formula Ia

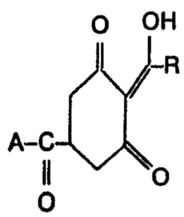

(Ia)

wherein A and R are as defined in claim 1, or a metal or ammonium salt thereof.

3. Cyclohexanedionecarboxylic acid derivatives according to claim 2, wherein A is an $-OR_2$ radical and R and $R_2$ are as defined in claim 1, or a metal or ammonium salt thereof.

4. Cyclohexanedionecarboxylic acid derivatives according to claim 2, wherein A is an $-NR_3R_4$ radical and R, $R_3$ and $R_4$ are as defined in claim 1, or a metal or ammonium salt thereof.

5. Cyclohexanedionecarboxylic acid derivatives according to claim 2, wherein A is as defined in claim 1 and R is a $C_3$–$C_6$cycloalkyl radical, or a metal or ammonium salt thereof.

6. Cyclohexanedionecarboxylic acid derivatives according to claim 1 of the formula Ib

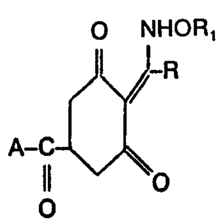

(Ib)

wherein A, R and $R_1$ are as defined in claim 1, or a metal or quaternary ammonium salt thereof.

7. Cyclohexanedionecarboxylic acid derivatives according to claim 6, wherein A is an $-OR_2$ radical and R, $R_1$ and $R_2$ are as defined in claim 1, or a metal or quaternary ammonium salt thereof.

8. Cyclohexanedionecarboxylic acid derivatives according to claim 6, wherein A is an $-NR_3R_4$ radical and R, $R_1$, $R_3$ and $R_4$ are as defined in claim 1, or a metal or quaternary ammonium salt thereof.

9. Ethyl 4-butyryl-3,5-cyclohexanedionecarboxylate according to claim 2.

10. Isobutyl 4-butyryl-3,5-cyclohexanedionecarboxylate according to claim 2.

11. Ethyl 4-(cyclopropylhydroxymethylidene)-3,5-cyclohexanedionecarboxylate according to claim 2.

12. Dimethyl 4-(1-ethoxyaminobutylidene)-3,5-cyclohexanedionecarboxamide according to claim 6.

13. Diethyl 4-(1-allyloxyaminobutylidene)-3,5-cyclohexanedionecarboxamide according to claim 6.

14. Benzyl 4-(1-ethoxyaminobutylidene)-3,5-cyclohexanedionecarboxamide according to claim 6.

15. Isobutyl 4-(1-allyloxyaminobutylidene)-3,5-cyclohexanedionecarboxylate according to claim 6.

16. A process for the preparation of cyclohexanedionecarboxylic acid derivatives of the formula I according to claim 1, which comprises reacting a 3,5-cyclohexanedionecarboxylic acid derivative of the formula II

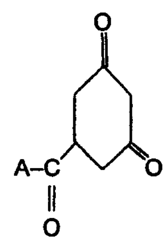

(II)

wherein A is an ester or amide radical as defined in claim 1, with an acid halide of the formula III

Hal–COR (III)

wherein R is as defined in claim 1, in an inert organic solvent and in the presence of a base as acid acceptor, isolating the product so obtained and, if desired, reacting it further with a hydroxylamine of the formula IV

$HONHR_1$ (IV)

wherein $R_1$ is as defined above, in an inert organic solvent at boiling temperature under condensation conditions, and isolating the resultant product.

17. A herbicidal and plant-growth-regulating composition, which contains, as active ingredient, at least one cyclohexanedionecarboxylic acid derivative of the formula I according to claim 1, together with carriers and/or other adjuvants.

18. The use of the cyclohexanedionecarboxylic acid derivatives of the formula I according to claim 1, or of compositions containing such compounds, for controlling undesirable plant growth.

19. The use of the cyclohexanedionecarboxylic acid derivatives of the formula I according to claim 1, or of compositions containing such compounds, for regulating plant growth.

20. The use of the cyclohexanedionecarboxylic acid derivatives of the formula I according to claim 1, or of compositions containing such compounds, for inhibiting the growth of grasses.

21. A method of selectively controlling weeds pre- or postemergence in crops of useful plants, which comprises treating the useful plants or the locus thereof with an effective amount of a cyclohexanedionecarboxylic acid derivative of the formula I according to claim 1, or of a composition containing such a derivative.

22. A method of selectively controlling weed grasses pre- and postemergence in crops of useful plants, which comprises treating the useful plants or the locus thereof with an effective amount of a cyclohexanedionecarboxylic acid derivative of the formula I according to claim 1, or of a composition containing such a derivative.

23. A method of regulating plant growth, which comprises treating plants, parts of plants or seeds with an effective amount of a cyclohexanedionecarboxylic acid ester of the formula I according to claim 1, or of a composition containing such a derivative.

24. The use of the cyclohexanedionecarboxylic acid derivatives of the formula I, or of compositions containing such compounds, for inhibiting plant growth beyond the 2-leaf stage, wherein the active ingredients are applied pre-emergence.

25. The use according to claim 22 in crops of sugar cane, cereals, maize, soybeans, rice and cotton.

26. The use of the cyclohexanedionecarboxylic acid derivatives of the formula I according to claim 1, or of compositions containing such compounds, for regulating the growth of cultivated plants for the purpose of increasing yield.

27. The use according to claim 26 in crops of soybeans.

28. The use according to claim 24 in leguminous cover crops.

**Claims for the Contracting State: AT**

1. A herbicidal and plant-growth-regulating composition, which contains, together with carriers and/or other adjuvants, at least one cyclohexanedionecarboxylic acid derivative of the formula I

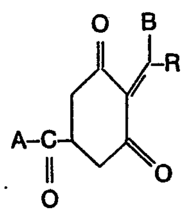

(I)

wherein

A is an $-OR_2$ or $-NR_3R_4$ radical,

B is hydroxyl or an $-NHOR_1$ radical,

R is $C_1$–$C_6$alkyl or $C_3$–$C_6$cycloalkyl, each unsubstituted or substituted by halogen, $C_1$–$C_4$alkoxy or $C_1$–$C_4$alkylthio,

$R_1$ is $C_1$–$C_6$alkyl, $C_1$–$C_6$haloalkyl, $C_3$–$C_6$alkenyl, $C_3$–$C_6$haloalkenyl or $C_3$–$C_6$alkynyl,

$R_2$, $R_3$ and $R_4$ are each independently hydrogen, $C_1$–$C_6$alkyl, $C_1$–$C_6$haloalkyl, $C_2$–$C_{10}$alkoxyalkyl, $C_2$–$C_{10}$alkylthioalkyl; $C_3$–$C_6$alkenyl which is unsubstituted or substituted by halogen, $C_1$–$C_4$alkoxy or $C_1$–$C_4$alkylthio; $C_3$–$C_6$alkynyl; phenyl or $C_1$–$C_6$aralkyl, wherein the phenyl nucleus is unsubstituted or substituted by halogen, $C_1$–$C_4$alkyl, $C_1$–$C_4$alkoxy, $C_1$–$C_4$haloalkyl, nitro or cyano, and

$R_3$ and $R_4$, together with the nitrogen atom to which they are bonded, also form a 5- or 6-membered heterocyclic ring system which may also contain an oxygen or sulfur atom in the ring,

or a metal or ammonium salt of such a cyclohexanedionecarboxylic acid derivative.

2. A herbicidal and plant-growth-regulating composition according to claim 1, which contains a cyclohexanedionecarboxylic acid derivative of the formula Ia

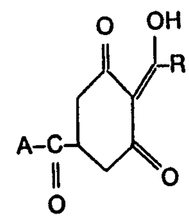

(Ia)

wherein A and R are as defined in claim 1, or a metal or ammonium salt thereof.

3. A herbicidal and plant-growth-regulating composition according to claim 2, which contains a cyclohexanedionecarboxylic acid derivative of the formula Ia wherein A is an $-OR_2$ radical and R and $R_2$ are as defined in claim 1, or a metal or ammonium salt thereof.

4. A herbicidal and plant-growth-regulating composition according to claim 2, which contains a cyclohexanedionecarboxylic acid derivative of the formula Ia wherein A is an $-NR_3R_4$ radical and R, $R_3$ and $R_4$ are as defined in claim 1, or a metal or ammonium salt thereof.

5. A herbicidal and plant-growth-regulating composition according to claim 2, which contains a cyclohexanedionecarboxylic acid derivative of the formula Ia wherein A is as defined in claim 1 and R is a $C_3$–$C_6$cycloalkyl radical, or a metal or ammonium salt thereof.

6. A herbicidal and plant-growth-regulating composition according to claim 1, which contains a cyclohexanedionecarboxylic acid derivative of the formula Ib

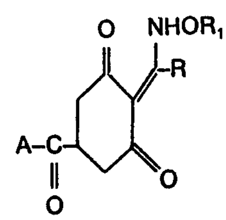

(Ib)

wherein A, R and $R_1$ are as defined in claim 1, or a metal or quaternary ammonium salt thereof.

7. A herbicidal and plant-growth-regulating composition according to claim 6, which contains a cyclohexanedionecarboxylic acid derivative of the formula Ib wherein A is an $-OR_2$ radical and R, $R_1$ and $R_2$ are as defined in claim 1, or a metal or quaternary ammonium salt thereof.

8. A herbicidal and plant-growth-regulating composition according to claim 6, which contains a cyclohexanedionecarboxylic acid derivative of the formula Ib wherein A is an $-NR_3R_4$ radical and R, $R_1$, $R_3$ and $R_4$ are as defined in claim 1, or a metal or quaternary ammonium salt thereof.

9. A herbicidal and plant-growth-regulating composition according to claim 2, which contains ethyl 4-butyryl-3,5-cyclohexanedionecarboxylate.

10. A herbicidal and plant-growth-regulating composition according to claim 2, which contains isobutyl 4-butyryl-3,5-cyclohexanedionecarboxylate.

11. A herbicidal and plant-growth-regulating composition according to claim 2, which contains ethyl 4-(cyclopropylhydroxymethylidene)-3,5-cyclohexanedionecarboxylate.

12. A herbicidal and plant-growth-regulating composition according to claim 6, which contains dimethyl 4-(1-ethoxyaminobutylidene)-3,5-cyclohexanedionecarboxamide.

13. A herbicidal and plant-growth-regulating composition according to claim 6, which contains diethyl 4-(allyloxyaminobutylidene)-3,5-cyclohexanedionecarboxamide.

14. A herbicidal and plant-growth-regulating composition according to claim 6, which contains benzyl 4-(1-ethoxyaminobutylidene)-3,5-cyclohexanedionecarboxamide.

15. A herbicidal and plant-growth-regulating composition according to claim 6, which contains isobutyl 4-(1-allyloxyaminobutylidene)-3,5-cyclohexanedionecarboxylate.

16. A process for the preparation of cyclohexanedionecarboxylic acid derivatives of the formula I according to claim 1, which comprises reacting a 3,5-cyclohexanedionecarboxylic acid derivative of the formula II

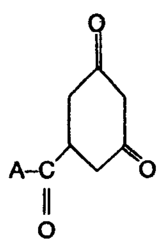

(II).

wherein A is an ester or amide radical as defined in claim 1, with an acid halide of the formula III

$$Hal-COR \qquad (III)$$

wherein R is as defined in claim 1, in an inert organic solvent and in the presence of a base as acid acceptor, isolating the product so obtained and, if desired, reacting it further with a hydroxylamine of the formula IV

$$HONHR_1 \qquad (IV)$$

wherein $R_1$ is as defined above, in an inert organic solvent at boiling temperature under condensation conditions, and, if desired, converting the resultant product into a metal or ammonium salt.

17. The use of the cyclohexanedionecarboxylic acid derivatives of the formula I according to claim 1, or of compositions containing such compounds, for controlling undesirable plant growth.

18. The use of the cyclohexanedionecarboxylic acid derivatives of the formula I according to claim 1, or of compositions containing such compounds, for regulating plant growth.

19. The use of the cyclohexanedionecarboxylic acid derivatives of the formula I according to claim 1, or of compositions containing such compounds, for inhibiting the growth of grasses.

20. A method of selectively controlling weeds pre- or postemergence in crops of useful plants, which comprises treating the useful plants or the locus thereof with an effective amount of a cyclohexanedionecarboxylic acid derivative of the formula I according to claim 1, or of a composition containing such a derivative.

21. A method of selectively controlling weed grasses pre- and postemergence in crops of useful plants, which comprises treating the useful plants or the locus thereof with an effective amount of a cyclohexanedionecarboxylic acid derivative of the formula I according to claim 1, or of a composition containing such a derivative.

22. A method of regulating plant growth, which comprises treating plants, parts of plants or seeds with an effective amount of a cyclohexanedionecarboxylic acid ester of the formula I according to claim 1, or of a composition containing such a derivative.

23. The use of the cyclohexanedionecarboxylic acid derivatives of the formula I, or of compositions containing such compounds, for inhibiting plant growth beyond the 2-leaf stage, wherein the active ingredients are applied pre-emergence.

24. The use according to claim 21 in crops of sugar cane, cereals, maize, soybeans, rice and cotton.

25. The use of the cyclohexanedionecarboxylic acid derivatives of the formula I according to claim 1, or of compositions containing such compounds, for regulating the growth of cultivated plants for the purpose of increasing yield.

26. The use according to claim 25 in crops of soybeans.

27. The use according to claim 23 in leguminous cover crops.

**Revendications pour les Etats contractants: BE, LU, NL, SE**

1. Dérivés d'acides cyclohexane-dione-carboxyliques de formule I

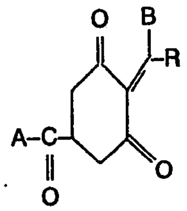

(I),

dans laquelle

A représente un groupe $-OR_2$ ou $-NR_3R_4$,

B représente un groupe hydroxy, un groupe $-NHOR_1$,

R représente un groupe alkyle en C 1–C 6 ou cycloalkyle en C 3–C 6, non substitué ou substitué par des halogènes, des groupes alcoxy en C 1–C 4 ou alkylthio en C 1–C 4,

$R_1$ représente un groupe alkyle en C 1–C 6, halogénoalkyle en C 1–C 6, alcényle en C 3–C 6, halogénoalcényle en C 3–C 6 ou alcynyle en C 3–C 6,

$R_2$, $R_3$ et $R_4$ représentent chacun, indépendamment les uns des autres, l'hydrogène; un groupe alkyle en C 1–C 6, halogénoalkyle en C 1–C 6, alcoxyalkyle en C 2–C 10, alkylthioalkyle en C 2–C 10; un groupe alcényle en C 3–C 6 non substitué ou substitué par des halogènes, des groupes alcoxy en C 1–C 4 ou alkylthio en C 1–C 4, un groupe alcynyle en C 3–C 6; un groupe phényle ou aralkyle en C 1–C 6 dans lequel le noyau phényle est non substitué ou substitué par des halogènes, des groupes alkyle en C 1–C 4, alcoxy en C 1–C 4, halogénoalkyle en C 1–C 4, nitro ou cyano,

$R_3$ et $R_4$ peuvent également former avec l'atome d'azote auquel ils sont reliés un hétérocycle à 5 ou 6 chaînons qui peut encore contenir, dans le cycle, un atome d'oxygène ou de soufre,

ou un sel de métal ou d'ammonium d'un tel dérivé d'acide cyclohexane-dione-carboxylique.

2. Dérivés d'acides cyclohexane-dione-carboxyliques selon la revendication 1, répondant à la formule Ia

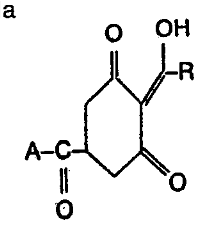

(Ia),

dans laquelle A et R ont les significations indiquées dans la revendication 1, ou un sel de métal ou d'ammonium d'un tel composé.

3. Dérivés d'acides cyclohexane-dione-carboxyliques selon la revendication 2, dans lesquels A représente un groupe $-OR_2$ et R et $R_2$ ont les significations indiquées dans la revendication 1, ou un sel de métal ou d'ammonium d'un tel composé.

4. Dérivés d'acides cyclohexane-dione-carboxyliques selon la revendication 2, dans lesquels A représente un groupe $-NR_3R_4$ et R, $R_3$ et $R_4$ ont les significations indiquées dans la revendication 1, ou un sel de métal ou d'ammonium d'un tel composé.

5. Dérivés d'acides cyclohexane-dione-carboxyliques selon la revendication 2, dans lesquels A a les significations indiquées dans la revendication 1 et R représente un groupe cycloalkyle en C 3–C 6, ou un sel de métal ou d'ammonium d'un tel composé.

6. Dérivés d'acides cyclohexane-dione-carboxyliques selon la revendication 1, répondant à la formule Ib

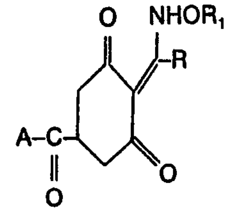

(Ib)

dans laquelle A, R et $R_1$ ont les significations indiquées dans la revendication 1, ou un sel de métal ou d'ammonium quaternaire d'un tel composé.

7. Dérivés d'acides cyclohexane-dione-carboxyliques selon la revendication 6, dans lesquels A représente un groupe $OR_2$ et R, $R_1$ et $R_2$ ont les significations indiquées dans la revendication 1, ou un sel de métal ou d'ammonium quaternaire d'un tel composé.

8. Dérivés d'acides cyclohexane-dione-carboxyliques selon la revendication 6, dans lesquels A représente un groupe $-NR_3R_4$ et R, $R_1$, $R_3$ et $R_4$ ont les significations indiquées dans la revendication 1, ou un sel de métal ou d'ammonium quaternaire d'un tel composé.

9. Le 4-butyryl-3,5-cyclohexane-dione-carboxylate d'éthyle selon la revendication 2.

10. Le 4-butyryl-3,5-cyclohexane-dione-carboxylate d'isobutyle selon la revendication 2.

11. Le 4-(cyclopropyl-hydroxyméthylidène)-3,5-cyclohexane-dione-carboxylate d'éthyle selon la revendication 2.

12. Le diméthylamide de l'acide 4-(1-éthoxyaminobutylidène)-3,5-cyclohexane-dione-carboxylique selon la revendication 6.

13. Le diéthylamide de l'acide 4-(1-allyloxyaminobutylidène)-3,5-cyclohexane-dione-carboxylique selon la revendication 6.

14. Le benzylamide de l'acide 4-(1-éthoxyaminobutylidène)-3,5-cyclohexane-dione-carboxylique selon la revendication 6.

15. Le 4-(1-allyloxyaminobutylidène)-3,5-cyclohexane-dione-carboxylate d'isobutyle selon la revendication 6.

16. Procédé de préparation des dérivés d'acides cyclohexane-dione-carboxyliques de formule I selon la revendication 1, caractérisé en ce que l'on fait réagir un dérivé d'acide 3,5-cyclohexane-dione-carboxylique de formule II

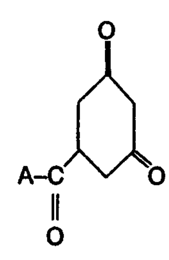

(II),

dans laquelle A représente un groupe ester ou amide tel que défini dans la revendication 1, dans un solvant organique inerte, en présence d'une base servant d'agent neutralisant les acides, avec un halogénure d'acide de formule III

$$\text{Hal–COR} \qquad \text{(III)},$$

dans laquelle R a les significations indiquées dans la revendication 1, on isole le produit obtenu et le cas échéant on le fait réagir dans un solvant organique inerte, à la température d'ébullition, dans des conditions propres à une élimination d'eau, avec une hydroxylamine de formule IV

$$HONHR_1 \qquad \text{(IV)}$$

dans laquelle $R_1$ a les significations indiquées ci-dessus, puis on isole le produit obtenu.

17. Un produit herbicide et régulateur de la croissance des végétaux, caractérisé en ce qu'il contient en tant que substance active, avec des véhicules et/ou d'autres additifs, au moins un dérivé d'acide cyclohexane-dione-carboxylique de formule I selon la revendication 1.

18. Utilisation des dérivés d'acides cyclohexane-dione-carboxyliques de formule I selon la revendication 1 ou de produits en contenant dans la lutte contre les croissances de végétaux indésirables.

19. L'utilisation des dérivés d'acides cyclohexane-dione-carboxyliques de formule I selon la revendication 1 ou de produits en contenant pour la régulation de la croissance des végétaux.

20. L'utilisation des dérivés d'acides cyclohexane-dione-carboxyliques de formule I selon la revendication 1 ou de produits en contenant pour l'inhibition de la croissance des graminées.

21. Procédé pour la lutte sélective en pré-levée ou post-levée contre les végétaux adventices dans les cultures de végétaux utiles, caractérisé en ce que l'on traite les végétaux utiles ou leur aire de culture par une quantité efficace d'un dérivé d'acide cyclohexane-dione-carboxylique de formule I selon la revendication 1 ou d'un produit contenant un tel dérivé.

22. Procédé pour la lutte sélective en pré-levée et post-levée contre les mauvaises herbes dans les cultures de végétaux utiles, caractérisé en ce que l'on traite les végétaux utiles ou leur aire de culture par une quantité efficace d'un dérivé d'acide cyclohexane-dione-carboxylique de formule I selon la revendication 1 ou d'un produit contenant un tel dérivé.

23. Procédé pour la régulation de la croissance des végétaux, caractérisé en ce que l'on traite les végétaux, des parties des végétaux ou les semences par une quantité efficace d'un ester d'acide cyclohexane-dione-carboxylique de formule I selon la revendication 1 ou d'un produit contenant un tel dérivé.

24. L'utilisation des dérivés d'acides cyclohexane-dione-carboxyliques de formule I ou de produits en contenant pour l'inhibition de la croissance des végétaux au-delà du stade 2 feuilles, caractérisée en ce que l'on applique les substances actives en pré-levée.

25. L'utilisation selon la revendication 22, dans les cultures de canne à sucre, de céréales, de maïs, de soja, de riz et de coton.

26. L'utilisation des dérivés d'acides cyclohexane-dione-carboxyliques de formule I selon la revendication 1 ou de produits en contenant pour la régulation de la croissance des végétaux cultivés en vue d'une augmentation du rendement.

27. L'utilisation selon la revendication 26, dans les cultures de soja.

28. L'utilisation selon la revendication 24, sur les légumineuses de couvertures de sol.

**Revendications pour les Etats contractants: CH, DE, FR, GB, IT, LI**

1. Dérivés d'acides cyclohexane-dione-carboxyliques de formule I

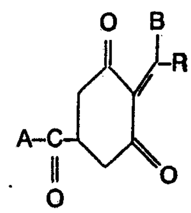

(I),

dans laquelle

A représente un groupe $-OR_2$ ou $-NR_3R_4$,

B représente un groupe hydroxy, un groupe $-NHOR_1$,

R représente un groupe alkyle en C 1–C 6 ou cycloalkyle en C 3–C 6, non substitué ou substitué par des halogènes, des groupes alcoxy en C 1–C 4 ou alkylthio en C 1–C 4,

$R_1$ représente un groupe alkyle en C 1–C 6, halogénoalkyle en C 1–C 6, alcényle en C 3–C 6, halogénoalcényle en C 3–C 6 ou alcynyle en C 3–C 6,

$R_2$, $R_3$ et $R_4$ représentent chacun, indépendamment les uns des autres, l'hydrogène; un groupe alkyle en C 1–C 6, halogénoalkyle en C 1–C 6, alcoxyalkyle en C 2–C 10, alkylthioalkyle en C 2–C 10; un groupe alcényle en C 3–C 6 non substitué ou substitué par des halogènes, des groupes alcoxy en C 1–C 4 ou alkylthio en C 1–C 4, un groupe alcynyle en C 3–C 6; un groupe phényle ou aralkyle en C 1–C 6 dans lequel le noyau phényle est non substitué ou substitué par des halogénes, des groupes alkyle en C 1–C 4, alcoxy en C 1–C 4, halogénoalkyle en C 1–C 4, nitro ou cyano,

$R_3$ et $R_4$ peuvent également former avec l'atome d'azote auquel ils sont reliés un hétérocycle à 5 ou 6 chaînons qui peut encore contenir, dans le cycle, un atome d'oxygène ou de soufre,

ou un sel de métal ou d'ammonium d'un tel dérivé d'acide cyclohexane-dione-carboxylique, étant spécifié que dans le cas des sels, R ne peut représenter un groupe alkyle en C 1–C 6 lorsque $R_2$ représente l'hydrogène ou un groupe alkyle en C 1–C 6.

2. Dérivés d'acides cyclohexane-dione-carboxyliques selon la revendication 1, répondant à la formule Ia

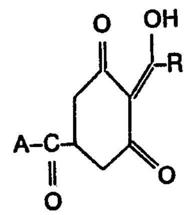

(Ia),

dans laquelle A et R ont les significations indiquées dans la revendication 1, ou un sel de métal ou d'ammonium d'un tel composé.

3. Dérivés d'acides cyclohexane-dione-carboxyliques selon la revendication 2, dans lesquels A représente un groupe $-OR_2$ et R et $R_2$ ont les significations indiquées dans la revendication 1, ou un sel de métal ou d'ammonium d'un tel composé.

4. Dérivés d'acides cyclohexane-dione-carboxyliques selon la revendication 2, dans lesquels A représente un groupe $-NR_3R_4$ et R, $R_3$ et $R_4$ ont les significations indiquées dans la revendication 1, ou un sel de métal ou d'ammonium d'un tel composé.

5. Dérivés d'acides cyclohexane-dione-carboxyliques selon la revendication 2, dans lesquels A a les significations indiquées dans la revendication 1 et R représente un groupe cycloalkyle en C 3–C 6, ou un sel de métal ou d'ammonium d'un tel composé.

6. Dérivés d'acides cyclohexane-dione-carboxyliques selon la revendication 1, répondant à la formule Ib

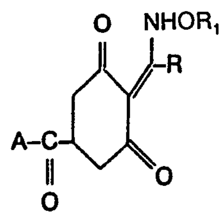

(Ib)

dans laquelle A, R et $R_1$ ont les significations indiquées dans la revendication 1, ou un sel de métal ou d'ammonium quaternaire d'un tel composé.

7. Dérivés d'acides cyclohexane-dione-carboxyliques selon la revendication 6, dans lesquels A représente un groupe $OR_2$ et R, $R_1$ et $R_2$ ont les significations indiquées dans la revendication 1, ou un sel de métal ou d'ammonium quaternaire d'un tel composé.

8. Dérivés d'acides cyclohexane-dione-carboxyliques selon la revendication 6, dans lesquels A représente un groupe $-NR_3R_4$ et R, $R_1$, $R_3$ et $R_4$ ont les significations indiquées dans la revendication 1, ou un sel de métal ou d'ammonium quaternaire d'un tel composé.

9. Le 4-butyryl-3,5-cyclohexane-dione-carboxylate d'éthyle selon la revendication 2.

10. Le 4-butyryl-3,5-cyclohexane-dione-carboxylate d'isobutyle selon la revendication 2.

11. Le 4-(cyclopropyl-hydroxyméthylidène)-3,5-cyclohexane-dione-carboxylate d'éthyle selon la revendication 2.

12. Le diméthylamide de l'acide 4-(1-éthoxyaminobutylidène)-3,5-cyclohexane-dione-carboxylique selon la revendication 6.

13. Le diéthylamide de l'acide 4-(1-allyloxyaminobutylidène)-3,5-cyclohexane-dione-carboxylique selon la revendication 6.

14. Le benzylamide de l'acide 4-(1-éthoxyaminobutylidène)-3,5-cyclohexane-dione-carboxylique selon la revendication 6.

15. Le 4-(1-allyloxyaminobutylidène)-3,5-cyclohexane-dione-carboxylate d'isobutyle selon la revendication 6..

16. Procédé de préparation des dérivés d'acides cyclohexane-dione-carboxyliques de formule I selon la revendication 1, caractérisé en ce que l'on fait réagir un dérivé d'acide 3,5-cyclohexane-dione-carboxylique de formule II

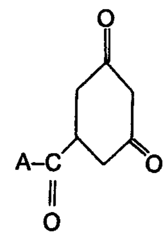

(II),

dans laquelle A représente un groupe ester ou amide tel que défini dans la revendication 1, dans un solvant organique inerte, en présence d'une base servant d'agent neutralisant les acides, avec un halogénure d'acide de formule III

Hal–COR (III),

dans laquelle R a les significations indiquées dans la revendication 1, on isole le produit obtenu et le cas échéant on le fait réagir dans un solvant organique inerte, à la température d'ébullition, dans des conditions propres à une élimination d'eau, avec une hydroxylamine de formule IV

$HONHR_1$ (IV),

dans laquelle $R_1$ a les significations indiquées ci-dessus, puis on isole le produit obtenu.

17. Un produit herbicide et régulateur de la croissance des végétaux, caractérisé en ce qu'il contient en tant que substance active, avec des véhicules et/ou d'autres additifs, au moins un dérivé d'acide cyclohexane-dione-carboxylique de formule I selon la revendication 1.

18. Utilisation des dérivés d'acides cyclohexane-dione-carboxyliques de formule I selon la revendication 1 ou de produits en contenant dans la lutte contre les croissances de végétaux indésirables.

19. L'utilisation des dérivés d'acides cyclohexane-dione-carboxyliques de formule I selon la revendication 1 ou de produits en contenant pour la régulation de la croissance des végétaux.

20. L'utilisation des dérivés d'acides cyclohexane-dione-carboxyliques de formule I selon la revendication 1 ou de produits en contenant pour l'inhibition de la croissance des graminées.

21. Procédé pour la lutte sélective en pré-levée ou post-levée contre les végétaux adventices dans les cultures de végétaux utiles, caractérisé en ce que l'on traite les végétaux utiles ou leur aire de culture par une quantité efficace d'un dérivé d'acide cyclohexane-dione-carboxylique de formule I selon la revendication 1 ou d'un produit contenant un tel dérivé.

22. Procédé pour la lutte sélective en pré-levée et post-levée contre les mauvaises herbes dans les cultures de végétaux utiles, caractérisé en ce que l'on traite les végétaux utiles ou leur aire de culture par une quantité efficace d'un dérivé d'acide cyclohexane-dione-carboxylique de formule I selon la revendication 1 ou d'un produit contenant un tel dérivé.

23. Procédé pour la régulation de la croissance des végétaux, caractérisé en ce que l'on traite les végétaux, des parties des végétaux ou les semences par une quantité efficace d'un ester d'acide cyclohexane-dione-carboxylique de formule I selon la revendication 1 ou d'un produit contenant un tel dérivé.

24. L'utilisation des dérivés d'acides cyclohexane-dione-carboxyliques de formule I ou de produits en contenant pour l'inhibition de la croissance des végétaux au-delà du stade 2 feuilles, caractérisée en ce que l'on applique les substances actives en pré-levée.

25. L'utilisation selon la revendication 22, dans les cultures de canne à sucre, de céréales, de maïs, de soja, de riz et de coton.

26. L'utilisation des dérivés d'acides cyclohexane-dione-carboxyliques de formule I selon la revendication 1 ou de produits en contenant pour la régulation de la croissance des végétaux cultivés en vue d'une augmentation du rendement.

27. L'utilisation selon la revendication 26, dans les cultures de soja.

28. L'utilisation selon la revendication 24, sur les légumineuses de couvertures de sol.

**Revendications pour l'Etat contractant: AT**

1. Produit herbicide et régulateur de la croissance des végétaux, caractérisé en ce qu'il contient, avec des véhicules et/ou d'autres additifs, au moins un dérivé d'acide cyclohexane-dione-carboxylique de formule I

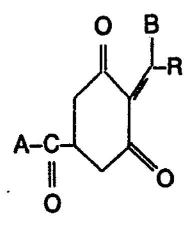

(I),

dans laquelle

A représente un groupe $-OR_2$ ou $-NR_3R_4$,

B représente un groupe hydroxy ou un groupe $-NHOR_1$,

R représente un groupe alkyle en C 1–C 6 ou cycloalkyle en C 3–C 6, non substitué ou substitué par des halogènes, des groupes alcoxy en C 1–C 4 ou alkylthio en C 1–C 4,

$R_1$ représente un groupe alkyle en C 1–C 6, halogénoalkyle en C 1–C 6, alcényle en C 3–C 6, halogénoalcényle en C 3–C 6 ou alcynyle en C 3–C 6,

$R_2$, $R_3$ et $R_4$ représentent chacun, indépendamment les uns des autres, l'hydrogène; un groupe alkyle en C 1–C 6, halogénoalkyle en C 1–C 6, alcoxyalkyle en C 2–C 10, alkylthioalkyle en C 2–C 10; un groupe alcényle en C 3–C 6 non substitué ou substitué par des halogénes, des groupes alcoxy en C 1–C 4 ou alkylthio en C 1–C 4; un groupe alcynyle en C 3–C 6; un groupe phényle ou aralkyle en C 1–C 6, le noyau phényle pouvant être non substitué ou substitué par des halogènes, des groupes alkyle en C 1–C 4, alcoxy en C 1–C 4, halogénoalkyle en C 1–C 4, nitro ou cyano,

$R_3$ et $R_4$ peuvent également former avec l'atome d'azote auquel ils sont reliés un groupe hétérocyclique de 5 à 6 chaînons qui peut encore contenir dans le cycle un atome d'oxygène ou de soufre, ou un sel de métal ou d'ammonium d'un tel dérivé d'acide cyclohexane-dione-carboxylique.

2. Produit herbicide et régulateur de la croissance des végétaux selon la revendication 1, caractérisé en ce qu'il contient un dérivé d'acide cyclohexane-dione-carboxylique de formule Ia

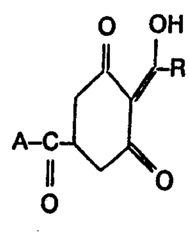

(Ia),

dans laquelle A et R ont les significations indiquées dans la revendication 1, ou un sel de métal ou d'ammonium d'un tel composé.

3. Produit herbicide et régulateur de la croissance des végétaux selon la revendication 2, caractérisé en ce qu'il contient un dérivé d'acide cyclohexane-dione-carboxylique de formule Ia dans laquelle A représente un groupe $-OR_2$ et R et $R_2$ ont les significations indiquées dans la revendication 1, ou un sel de métal ou d'ammonium d'un tel composé.

4. Produit herbicide et régulateur de la croissance des végétaux selon la revendication 2, caractérisé en ce qu'il contient un dérivé d'acide cyclohexane-dione-carboxylique de formule Ia dans laquelle A représente un groupe $-NR_3R_4$ et R, $R_3$ et $R_4$ ont les significations indiquées dans la revendication 1, ou un sel de métal ou d'ammonium d'un tel composé.

5. Produit herbicide et régulateur de la croissance des végétaux selon la revendication 2, caractérisé en ce qu'il contient un dérivé d'acide cyclohexane-dione-carboxylique de formule Ia dans laquelle A a les significations indiquées dans la revendication 1 et R représente un groupe cycloalkyle en C 3–C 6, ou un sel de métal ou d'ammonium d'un tel composé.

6. Produit herbicide et régulateur de la croissance des végétaux selon la revendication 1, caractérisé en ce qu'il contient un dérivé d'acide cyclohexane-dione-carboxylique de formule Ib

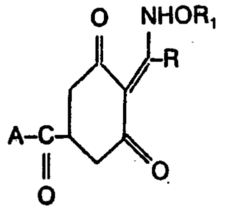

(Ib)

dans laquelle A, R et $R_1$ ont les significations indiquées dans la revendication 1, ou un sel de métal ou d'ammonium d'un tel composé.

7. Produit herbicide et régulateur de la croissance des végétaux selon la revendication 6, caractérisé en ce qu'il contient un dérivé d'acide cyclohexane-dione-carboxylique de formule Ib dans laquelle A représente un groupe $OR_2$ et R, $R_1$ et $R_2$ ont les significations indiquées dans la revendication 1, ou un sel de métal ou d'ammonium quaternaire d'un tel composé.

8. Produit herbicide et régulateur de la croissance des végétaux selon la revendication 6, caractérisé en ce qu'il contient un dérivé d'acide cyclohexane-dione-carboxylique de formule Ib dans laquelle A représente un groupe $-NR_3R_4$ et R, $R_1$, $R_3$ et $R_4$ ont les significations indiquées dans la revendication 1, ou un sel de métal ou d'ammonium quaternaire d'un tel composé.

9. Produit herbicide et régulateur de la croissance des végétaux selon la revendication 2, caractérisé en ce qu'il contient le 4-butyryl-3,5-cyclohexane-dione-carboxylate d'éthyle.

10. Produit herbicide et régulateur de la croissance des végétaux selon la revendication 2, caractérisé en ce qu'il contient le 4-butyryl-3,5-cyclohexane-dione-carboxylate d'isobutyle.

11. Produit herbicide et régulateur de la croissance des végétaux selon la revendication 2, caractérisé en ce qu'il contient le 4-(cyclopropylhydroxyméthylidène)-3,5-cyclohexane-dione-carboxylate d'éthyle.

12. Produit herbicide et régulateur de la croissance des végétaux selon la revendication 6, caractérisé en ce qu'il contient le diméthylamide de l'acide 4-(1-éthoxyaminobutylidène)-3,5-cyclohexane-dione-carboxylique.

13. Produit herbicide et régulateur de la croissance des végétaux selon la revendication 6, caractérisé en ce qu'il contient le diéthylamide de l'acide 4-(allyloxyaminobutylidène)-3,5-cyclohexane-dione-carboxylique.

14. Produit herbicide et régulateur de la croissance des végétaux selon la revendication 6, caractérisé en ce qu'il contient le benzylamide de l'acide 4-(1-éthoxyaminobutylidène)-3,5-cyclohexane-dione-carboxylique.

15. Produit herbicide et régulateur de la croissance des végétaux selon la revendication 6, caractérisé en ce qu'il contient le 4-(1-allyloxyaminobutylidène)-3,5-cyclohexane-dione-carboxylate d'isobutyle.

16. Procédé de préparation des dérivés d'acides cyclohexane-dione-carboxyliques de formule I selon la revendication 1, caractérisé en ce que l'on fait réagir un dérivé d'acide 3,5-cyclohexane-dione-carboxylique de formule II

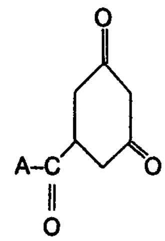

(II),

dans laquelle A représente un groupe ester ou amide tel que défini dans la revendication 1, dans un solvant organique inerte, en présence d'une base servant à neutraliser les acides, avec un halogénure d'acide de formule III

Hal–COR (III),

dans laquelle R a les significations indiquées dans la revendication 1, on isole le produit obtenu et le cas échéant on le fait réagir dans un solvant organique inerte, à la température d'ébullition, dans des conditions propres à une séparation d'eau, avec l'hydroxylamine de formule IV

$HONHR_1$ (IV),

dans laquelle $R_1$ a les significations indiquées ci-dessus, et si on le désire, on convertit le produit obtenu en un sel de métal ou d'ammonium.

17. L'utilisation des dérivés d'acides cyclohexane-dione-carboxyliques de formule I selon la revendication 1 ou de produits en contenant dans la lutte contre les croissances de végétaux indésirables.

18. L'utilisation des dérivés d'acides cyclohexane-dione-carboxyliques de formule I selon la revendication 1 ou de produits en contenant pour la régulation de la croissance des végétaux.

19. L'utilisation des dérivés d'acides cyclohexane-dione-carboxyliques de formule I selon la revendication 1 ou de produits en contenant pour l'inhibition de la croissance des graminées.

20. Procédé pour la lutte sélective en pré-levée ou post-levée contre les végétaux adventices dans les cultures de végétaux utiles, caractérisé en ce que l'on traite les végétaux utiles ou leur aire de culture par une quantité efficace d'un dérivé d'acide cyclohexane-dione-carboxylique de formule I selon la revendication 1 ou d'un produit contenant un tel dérivé.

21. Procédé pour combattre sélectivement en pré- et post-levée les mauvaises herbes dans les cultures de végétaux utiles, caractérisé en ce que l'on traite les végétaux utiles ou leur aire de culture par une quantité efficace d'un dérivé d'acide cyclohexane-dione-carboxylique de formule I selon la revendication 1 ou d'un produit contenant un tel dérivé.

22. Procédé pour la régulation de la croissance des végétaux, caractérisé en ce que l'on traite les végétaux, des parties des végétaux ou leurs semences par une quantité efficace d'un ester d'acide cyclohexane-dione-carboxylique de formule I selon la revendication 1 ou d'un produit contenant un tel dérivé.

23. L'utilisation des dérivés d'acides cyclohexane-dione-carboxyliques de formule I ou de produits en contenant pour l'inhibition de la croissance des végétaux au-delà du stade 2 feuilles, caractérisée en ce que l'on applique les substances actives en pré-levée.

24. L'utilisation selon la revendication 21, dans les cultures de canne à sucre, de céréales, de maïs, de soja, de riz et de coton.

25. L'utilisation des dérivés d'acides cyclohexane-dione-carboxyliques de formule I selon la revendication 1 ou de produits en contenant pour la régulation de la croissance des végétaux cultivés en vue d'une augmentation de rendement.

26. L'utilisation selon la revendication 25, dans les cultures de soja.

27. L'utilisation selon la revendication 23, sur les légumineuses de couvertures de sol.